# EUROPEAN PATENT APPLICATION

(11) **EP 4 748 417 A1**
(43) Date of publication of application: **27.05.2026**
(21) Application number: 24843022.5
(22) Date of filing: 09.07.2024
(51) Int. Cl.: A61M 5/32, A61M 25/06, A61M 5/158

(54) **NEEDLE TIP PROTECTOR**

(30) Priority: 20.07.2023 JP 2023118207
(71) Applicant: Nipro Corporation, Settsu-shi, Osaka 566-8510 (JP)
(72) Inventor: SAKAMOTO, Shingo, Settsu-shi, Osaka 566-8510 (JP)
(74) Representative: Slingsby Partners LLP
(86) International application number: PCT/JP2024/024799
(87) International publication number: WO 2025/018230

(57) **Abstract**

A needle tip protector 10 for preventing exposure of a needle tip 38 by moving to a distal end of the tip 38 of a puncture needle 34. Proximal ends of a pair of side plates 50, 52 extending along the needle 34 are mutually connected by a bottom part 48. When attached to the needle 34, the needle 34 inserted through a needle insertion hole 54 of the bottom part 48 is inserted between the plates 50, 52, and the plates 50, 52 are elastically deformed to sides of mutual separation. Needle-tip covers 76, 110 protruding inward in a direction of mutual facing of the plates 50, 52 are provided on distal ends of the plates 50, 52. Narrow parts 66, 100 of the plates 50, 52 each having a width of 70% or less of a width of the needle-tip covers 76, 110 is flexible, and the narrow parts 66, 100 each have a length of 50% or more of a length of the plates 50, 52 having the narrow parts 66, 100.

## Description

### TECHNICAL FIELD

This invention relates to a needle tip protector that protects the needle tip of a puncture needle after use.

### BACKGROUND ART

Conventionally, a needle tip protector has been known to be attached to a puncture needle to protect the needle tip of the puncture needle after use by moving toward the needle tip side. For example, the present applicant discloses a needle tip protector in U.S. Patent No. US 4,929,241 (Patent Document 1). The needle tip protector of Patent Document 1 includes a set of side plates extending to the needle tip side from the bottom part where the insertion hole for the puncture needle is provided, and the distal end portions of the set of side plates approach each other to form a needle-tip guarding part covering the needle tip.

### BACKGROUND ART DOCUMENTS

### PATENT DOCUMENTS

Patent Document 1: US 4,929,241

### SUMMARY OF THE INVENTION

### PROBLEM THE INVENTION ATTEMPTS TO SOLVE

However, as a result of further examination by the inventor into the needle tip protector of Patent Document 1, it became clear that there was still room for improvement.

For example, in the needle tip protector of Patent Document 1, when the needle tip protector moves to the needle tip side of the puncture needle, the needle-tip cover part constituting the needle-tip guarding part of the needle tip protector slides against the outer circumferential surface of the puncture needle. Here, it became clear that the said sliding contact resistance is relatively large, and a large force is required to pull out the puncture needle. In particular, in the case where a projection to be locked with the bottom part of the needle tip protector is provided on the outer circumferential surface of the distal end portion of the puncture needle, the resistance increases when the needle-tip cover part of the needle tip protector climbs over the said projection. Thus, it was necessary to further reduce the force required for pulling out the puncture needle in order to facilitate the pulling-out operation.

Besides, in the case where the needle tip protector attached to the puncture needle is inserted into the radial inside of the outer needle hub, the needle tip protector slides against the inner circumferential surface of the outer needle hub. Thus, it was conceivable that the sliding resistance makes the insertion operation of the needle tip protector into the outer needle hub difficult, or the inner circumferential surface of the outer needle hub is damaged by, for example, being scraped by the sliding contact of the needle tip protector.

In addition, for example, in the case where a plurality of needle tip protectors in the isolated state are stored and transported in a packaged state by being bottled or the like, the distal end portion of a needle tip protector may enter the space between the facing pair of side plates of another needle tip protector to be interlocked. In this case, for example, the operator needs to unlock those multiple needle tip protectors by hand, which makes the operation complicated.

Moreover, it is necessary for the needle insertion hole formed in the bottom part of the needle tip protector to allow the puncture needle to be inserted and to allow the minute protrusion on the distal end portion of the puncture needle to be locked. Thus, the needle insertion hole has a dimension that is approximately equal to and slightly larger than the outer diameter dimension of the puncture needle. Therefore, the alignment accuracy of the puncture needle and the needle insertion hole required when inserting the puncture needle through the needle insertion hole is high, which makes the insertion operation difficult.

Furthermore, in the needle tip protector attached to the puncture needle, the pair of side plates both extend approximately parallel to the needle axis, so that the width dimension in the direction of opposition of the pair of side plates is comparatively large at the proximal end portion. Therefore, for example, in the case where the inner needle hub is inserted into the outer needle hub at the outer circumference of the proximal end portion of the needle tip protector, the inner needle hub is likely to interfere with the proximal end portion of the needle tip protector.

It is therefore one object of the present invention to provide a needle tip protector of novel structure which is able to solve at least one of the problems of the needle tip protector described above.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, needle-tip cover parts are separately provided to respective distal end sides of the pair of side plates while protruding inward in a direction of mutual facing of the pair of side plates, a narrow part is provided to each of the pair of side plates, the narrow part having a width dimension that is not greater than 70% of a maximum width dimension of the corresponding needle-tip cover part while being flexible in the direction of mutual facing of the pair of side plates, and a length dimension of the narrow part is at least 50% of a length dimension of the corresponding side plate having the narrow part.

According to the needle tip protector structured following the present preferred embodiment, the narrow part, which is narrower than the needle-tip cover part, is provided with a sufficient length in the side plate. Thus, the side plate easily undergoes flexural deformation at the narrow part, and the contact pressure of the needle-tip cover part on the puncture needle in the pair of side plates can be reduced. Therefore, for example, when the needle tip protector moves to the needle tip side of the puncture needle by the puncture needle being pulled out, the resistance due to sliding contact between the needle-tip cover part provided at the distal end portion of the side plate and the puncture needle is decreased, and the puncture needle can be pulled out with less force. As can be seen from the above, the narrow part is a low-rigidity part whose deformation rigidity (flexural deformation rigidity) in the plate thickness direction is reduced by being narrow, and is a flexible portion. For example, in the case where the needle tip protector includes a lateral cover piece that covers the lateral side of the puncture needle formed by bending the side plate at its widthwise outside, the connection portion between the side plate and the lateral cover piece has high bending deformation rigidity due to the formation of the lateral cover part, and is restricted in its flexural deformation in the direction of mutual facing of the pair of side plates. Thus, the said connection portion does not fall under the narrow part.

In the side plates extending along the puncture needle on the opposite sides of the puncture needle, it is desirable that the narrow part be provided to be located in the widthwise middle portion of the side plates. Specifically, for example, widthwise opposite sides of the side plate are located closer to the widthwise center than the widthwise opposite sides of the bottom part, thereby forming the narrow part located in the widthwise middle portion of the side plate.

A second preferred embodiment provides the needle tip protector according to the first preferred embodiment, wherein in each needle-tip cover part, a length dimension of the needle-tip cover part is at least 50% of the maximum width dimension of the needle-tip cover part, and in the attached state to the puncture needle, an angle formed by a needle axis of the puncture needle and a straight line, the straight line connecting a point in the needle-tip cover part that is located outermost in the direction of mutual facing of the pair of side plates and a point of contact with the puncture needle, is not greater than 60 degrees.

According to the needle tip protector structured following the present preferred embodiment, the length dimension of the needle-tip cover part is long, and the angle formed by the needle axis direction and the straight line, which connects the point of the outermost diameter of the needle tip cover part and the contact point with the puncture needle in the attached state, is not greater than 60 degrees. Thus, the external force of the component in the axis-perpendicular direction on the puncture needle exerted by the elastic force of the needle tip protector can be reduced, and the sliding resistance when pulling out the puncture needle can be decreased. Besides, in the case where the needle tip protector attached to the puncture needle is inserted into the radial inside of the outer needle hub or the like, the needle tip protector is unlikely to be pressed strongly against the inner circumferential surface of the outer needle hub due to the displacement of the needle-tip cover part, and damage to the inner circumferential surface of the outer needle hub or the like due to the pressing of the needle tip protector is also prevented.

A third preferred embodiment provides the needle tip protector according to the first or second preferred embodiment, wherein lateral cover pieces are separately provided to respective widthwise edges of the pair of side plates and protrude inward in the direction of mutual facing, the respective widthwise edges being opposite to each other in a plate width direction, and a connection portion between the widthwise edge of each of the pair of side plates and the corresponding lateral cover piece has a connection width dimension that is not greater than 70% of a maximum width dimension of the corresponding lateral cover piece in a direction parallel to the connection width dimension.

According to the needle tip protector structured following the present preferred embodiment, the lateral cover pieces prevent the puncture needle from being exposed to the lateral side, thereby enhancing the needle-tip protection function.

Besides, the width dimension of the connection portion between the widthwise edge of the side plate and the lateral cover piece is limited, thereby suppressing the reinforcing action on the side plate by the lateral cover piece, and flexural deformation of the side plate is easily allowed. Thus, for example, ease of pulling out the puncture needle, ease of insertion into the outer needle hub, etc., avoidance of damage to the outer needle hub, etc. and the like are achieved.

A fourth preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, needle-tip cover parts are separately provided to respective distal end sides of the pair of side plates while protruding inward in a direction of mutual facing of the pair of side plates, in each needle-tip cover part, a length dimension of the needle-tip cover part is at least 50% of a maximum width dimension of the needle-tip cover part, and in the attached state to the puncture needle, an angle formed by a needle axis of the puncture needle and a straight line, the straight line connecting a point in the needle-tip cover part that is located outermost in the direction of mutual facing of the pair of side plates and a point of contact with the puncture needle, is not greater than 60 degrees.

According to the needle tip protector structured following the present preferred embodiment, the length dimension of the needle-tip cover part is long, and the angle formed by the needle axis direction and the line, which connects the point of the outermost diameter of the needle-tip cover part and the contact point with the puncture needle in the attached state, is not greater than 60 degrees. Thus, the external force of the component in the axis-perpendicular direction on the puncture needle exerted by the elastic force of the needle tip protector can be reduced, and the sliding resistance when pulling out the puncture needle can be decreased. Besides, for example, in the case where the needle tip protector attached to the puncture needle is inserted into the radial inside of the outer needle hub or the like, the needle tip protector is unlikely to be pressed strongly against the inner circumferential surface of the outer needle hub due to the displacement of the needle-tip cover part, and damage to the inner circumferential surface of the outer needle hub or the like due to the pressing of the needle tip protector is also prevented.

A fifth preferred embodiment provides the needle tip protector according to any one of the first through fourth preferred embodiments, wherein respective proximal end parts of pair of the needle-tip cover parts are located at positions different from each other in a needle axis direction of the puncture needle.

According to the needle tip protector structured following the present preferred embodiment, by mutually differentiating the positions of the proximal end parts of the needle-tip cover parts, degree of freedom in designing the shape including the needle-tip cover parts is increased. This makes it easy, for example, to adjust the length of each needle-tip cover part, to adjust the needle tip protection mode covering the needle tip, to adjust the engaging action of the needle-tip cover parts with the outer needle hub, and the like. Besides, for example, in the case where the needle-tip cover part extends to the distal end side while inclining toward the puncture needle, since the positions of the proximal end parts of the needle-tip cover parts are different in the needle axis direction, it is easy to position the distal end of one needle-tip cover part more distal end side with respect to the distal end of the other needle-tip cover part. By so doing, in the needle-tip protection state, one needle-tip cover part overlaps the other needle-tip cover part with a larger overlap margin, and more stable protection of the needle tip is achieved.

A sixth preferred embodiment provides the needle tip protector according to the fifth preferred embodiment, wherein an outer needle hub on a proximal end side of an outer needle configured to be externally placed about the puncture needle includes a housing part having a concave shape to house the pair of the needle-tip cover parts, and the respective proximal end parts of the pair of the needle-tip cover parts are both located on a straight inner circumferential surface of the housing part.

According to the present preferred embodiment, even if the needle tip protector has a structure in which the proximal end parts of the pair of needle-tip cover parts are arranged at different positions in the needle axis direction, by both the proximal end parts of those needle-tip cover parts being located on the straight inner circumferential surface of the housing part of the outer needle hub, the needle-tip cover parts are stably housed in the housing part of the outer needle hub without, for example, only one of the needle tip cover parts being pressed strongly against the outer needle hub.

A seventh preferred embodiment provides a needle tip protector for preventing exposure of a needle tip by being attached to a puncture needle and moving to a distal end side where the needle tip is located, comprising a pair of side plates extending along the puncture needle on opposite sides of the puncture needle, wherein in an attached state to the puncture needle, the puncture needle is inserted between the pair of side plates such that the pair of side plates are elastically deformed to sides of mutual separation, needle-tip cover parts are separately provided to respective distal end sides of the pair of side plates while protruding inward in a direction of mutual facing of the pair of side plates, and in an isolated state of the needle tip protector that is not attached to the puncture needle, a distance between facing surfaces of the pair of side plates is set to such a size that prevents entry of the needle-tip cover parts in initial shapes.

The needle-tip cover parts covering the needle tip are likely to be provided with steps in order to have such a shape that can effectively protect the needle tip. Thus, if the needle-tip cover parts enter the space between the facing surfaces of the pair of side plates of another needle tip protector, the said steps may be caught by the side plates or the like, thereby making them difficult to be separated. Therefore, in the needle tip protector structured following the present preferred embodiment, the distance between the facing surfaces of the pair of side plates is set to such a size that prevents entry of the needle-tip cover parts in initial shapes. With this configuration, for example, in the case where a plurality of needle tip protectors in the isolated state are housed in a bottle, etc. for storage, transportation, and the like, the needle-tip cover parts, which are the distal end portion of the needle tip protector, are unlikely to enter the space between the facing surfaces of the pair of side plates of another needle tip protector. Accordingly, it is possible to prevent the plurality of needle tip protectors housed in one container from being interlocked, thereby reducing the labor required to unlock the needle tip protectors.

An eighth preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, and in an isolated state of the needle tip protector that is not attached to the puncture needle, a relative inclination angle between respective proximal end portions of the pair of side plates is smaller than a relative inclination angle between respective distal end portions of the pair of side plates.

According to the needle tip protector structured following the present preferred embodiment, the ease of attachment of the needle tip protector to the outer needle hub can be improved and the size of the needle tip protector can be reduced. Specifically, for example, it is assumed that inner circumferential surface of the outer needle hub is provided with an engaging part with the needle tip protector. Here, when the needle tip protector is attached to the outer needle hub, in a structure where the side plates are extended to the distal end at a constant angle without increasing the width of the bottom part (the distance between the facing pair of side plates at the proximal end), the side plates, which were flexed by the stress caused by the needle tip protector climbing over the engaging part of the outer needle hub, may come into contact with the puncture needle, posing a risk of reducing the ease of attachment of the needle tip protector to the outer needle hub. On the other hand, in the case where the width of the bottom part is increased and the side plates are extended to the distal end at a constant angle, the needle tip protector becomes larger. Therefore, in the present preferred embodiment, in the isolated state of the needle tip protector that is not attached to the puncture needle, the relative inclination angle between the proximal end portions of the pair of side plates is smaller than the relative inclination angle between the distal end portions of the pair of side plates. This suppresses reduction in the ease of attachment of the needle tip protector to the outer needle hub and increase in the size of the needle tip protector in the state of being attached to the puncture needle.

A ninth preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, lateral cover pieces are separately provided to respective widthwise edges of the pair of side plates and protrude inward in a direction of mutual facing of the pair of side plates, the respective widthwise edges being opposite to each other in a plate width direction, and a connection portion between the widthwise edge of each of the pair of side plates and the corresponding lateral cover piece has a connection width dimension that is not greater than 70% of a maximum width dimension of the corresponding lateral cover piece.

According to the needle tip protector structured following the present preferred embodiment, the reinforcing action exerted on the side plate by providing the lateral cover piece is suppressed by the width dimension of the connection portion between the side plate and the lateral cover piece being limited, and flexural deformation of the side plate is easily allowed. Therefore, for example, when the needle tip protector moves to the needle tip side of the puncture needle by the puncture needle being pulled out, the resistance due to sliding contact between the needle-tip cover part provided at the distal end side of the side plate and the puncture needle is reduced, and the puncture needle can be pulled out with less force. Besides, since the maximum width dimension of the lateral cover piece is made large, it is possible to more effectively prevent exposure of the puncture needle to the lateral side. In particular, even if the position of the puncture needle with respect to the needle tip protector varies in the needle axis direction in the needle-tip protection state, the lateral side of the puncture needle is stably covered by the lateral cover piece, and the exposure of the puncture needle to the lateral side is prevented by the lateral cover piece.

A tenth preferred embodiment provides a needle tip protector for preventing exposure of a needle tip by being attached to a puncture needle and moving to a distal end side where the needle tip is located, comprising a bottom part having a plate shape including a needle insertion hole through which the puncture needle is inserted, wherein a needle guide surface having an inclination angle is provided to an opening peripheral edge of the needle insertion hole, and a pair of side plates having elasticity are provided from the bottom part toward the distal end side.

According to the needle tip protector structured following the present preferred embodiment, the needle guide surface is provided at the opening peripheral edge of the needle insertion hole. Thus, when inserting the puncture needle through the needle insertion hole, even if the puncture needle is slightly deviated in the radial direction with respect to the needle insertion hole, the puncture needle is guided to the needle insertion hole side by the needle guide surface, so that the puncture needle can be inserted through the needle insertion hole. In this way, the guiding action of the needle guide surface provided at the opening peripheral edge of the needle insertion hole moderates the required alignment accuracy between the puncture needle and the needle insertion hole, and facilitates the insertion of the puncture needle through the needle insertion hole. Note that the pair of side plates may extend along the puncture needle on the opposite sides of the puncture needle, or may extend toward the distal end so as to cross each other.

An eleventh preferred embodiment provides the needle tip protector according to the tenth preferred embodiment, wherein the needle guide surface is provided to the opening peripheral edge on one side of the needle insertion hole, and a reinforcing tube part having a tubular shape protrudes from an opening peripheral edge on an other side of the needle insertion hole.

According to the needle tip protector structured following the present preferred embodiment, the needle guide surface formed at the opening peripheral edge of one side of the needle insertion hole facilitates insertion of the puncture needle into the needle insertion hole. Besides, although there is a risk that the rigidity of the bottom part may be reduced by providing an inclination at the opening peripheral edge, the bottom part of the needle tip protector can be reinforced by the reinforcing tube part protruding from the opening peripheral edge on the other side of the needle insertion hole.

The needle guide surface and the reinforcing tube part are respectively provided at the opening peripheral edges on one and the other sides of the needle insertion hole. Thus, the needle guide surface and the reinforcing tube part can also be formed in a single step by, for example, performing burring process or pressing working on the peripheral edge of the needle insertion hole.

A twelfth preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, and in the attached state before protection of the needle tip, a relative inclination angle between respective proximal end portions of the pair of side plates is larger than a relative inclination angle between respective distal end portions of the pair of side plates.

According to the needle tip protector structured following the present preferred embodiment, it is possible to obtain a sufficient inclination angle on the distal end side of the needle tip protector while achieving compactization on the proximal end side of the needle tip protector.

A thirteenth preferred embodiment provides a needle tip protector for preventing exposure of a needle tip by being attached to a puncture needle and moving to a distal end side where the needle tip is located, comprising: a pair of side plates extending along the puncture needle on opposite sides of the puncture needle; and a bottom part connecting proximal end sides of the pair of side plates with each other, wherein in an attached state to the puncture needle, the puncture needle is inserted through a needle insertion hole penetrating the bottom part, while being inserted between the pair of side plates such that the pair of side plates are elastically deformed to sides of mutual separation, at least one of the pair of side plates includes a lateral cover piece protruding inward in a direction of mutual facing of the pair of side plates from a widthwise edge of the at least one of the pair of side plates, and a connection portion between the widthwise edge of the at least one of the pair of side plates and the lateral cover piece has a connection width dimension that is not greater than 70% of a maximum width dimension of the lateral cover piece.

According to the needle tip protector structured following the present preferred embodiment, the reinforcing action exerted on the side plate by providing the lateral cover piece is suppressed by the width dimension of the connection portion between the side plate and the lateral cover piece being limited, and flexural deformation of the side plate is easily allowed. Therefore, for example, when the needle tip protector moves to the needle tip side of the puncture needle by the puncture needle being pulled out, the resistance due to sliding contact between the needle-tip cover part provided at the distal end side of the side plate and the puncture needle is reduced, and the puncture needle can be pulled out with less force. Besides, since the maximum width dimension of the lateral cover piece is made large, it is possible to more effectively prevent exposure of the puncture needle to the lateral side. In particular, even if the position of the puncture needle with respect to the needle tip protector varies in the needle axis direction in the needle-tip protection state, the lateral side of the puncture needle is stably covered by the lateral cover piece, and the exposure of the puncture needle to the lateral side is prevented by the lateral cover piece.

### EFFECT OF THE INVENTION

According to the present invention, it is possible to attain at least one of the objects unsolved by conventional needle tip protectors such as, for example, reducing contact pressure against the puncture needle, the outer needle hub, and the like, preventing interlock in the isolated state, facilitating attachment to the puncture needle, and compactization in the distal end portion and avoidance of interference with the inner needle hub or the like in the proximal end portion in the attached state to the puncture needle.

### BRIEF DESCRIPTION OF THE DRAWINGS

[FIG. 1] is a perspective view showing an indwelling needle assembly including a needle tip protector according to a first practical embodiment of the present invention.
[FIG. 2] is a vertical cross sectional view of the indwelling needle assembly shown in FIG. 1, corresponding to a cross section taken along line 2-2 of FIG. 3.
[FIG. 3] is a cross sectional view taken along line 3-3 of FIG. 2.
[FIG. 4] is an enlarged view of a cross section taken along line 4-4 of FIG. 3.
[FIG. 5] is a perspective view of a needle tip protector constituting the indwelling needle assembly shown in FIG. 1.
[FIG. 6] is a perspective view of the needle tip protector shown in FIG. 5 from another angle.
[FIG. 7] is a top plan view of the needle tip protector shown in FIG. 5.
[FIG. 8] is a bottom plan view of the needle tip protector shown in FIG. 5.
[FIG. 9] is a front view of the needle tip protector shown in FIG. 5.
[FIG. 10] is a rear view of the needle tip protector shown in FIG. 5.
[FIG. 11] is a left side view of the needle tip protector shown in FIG. 5.
[FIG. 12] is a right side view of the needle tip protector shown in FIG. 5.
[FIG. 13] is a cross sectional view taken along line 13-13 of FIG. 9.
[FIG. 14] is a cross sectional view taken along line 14-14 of FIG. 11.
[FIG. 15] is a cross sectional view taken along line 15-15 of FIG. 11.
[FIG. 16] is a cross sectional view taken along line 16-16 of FIG. 9.
[FIG. 17] is a perspective view of the needle tip protector shown in FIG. 5 in an attached state to a puncture needle.
[FIG. 18] is a vertical cross sectional view of the needle tip protector shown in FIG. 5 in the attached state to the puncture needle.
[FIG. 19] is a vertical cross sectional view of the needle tip protector shown in FIG. 5 in a needle-tip protection state.
[FIG. 20] is a perspective view showing a needle tip protector according to a second practical embodiment of the present invention.
[FIG. 21] is a transverse cross sectional view of the needle tip protector shown in FIG. 20.

### EMBODIMENTS FOR CARRYING OUT THE INVENTION

Practical embodiments of the present invention will be described below in reference to the drawings.

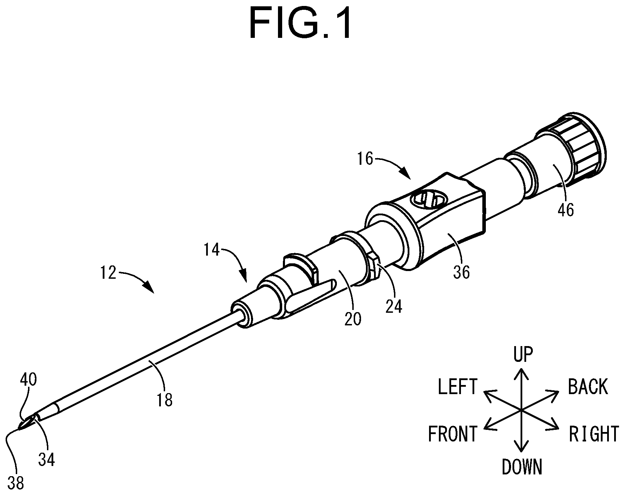

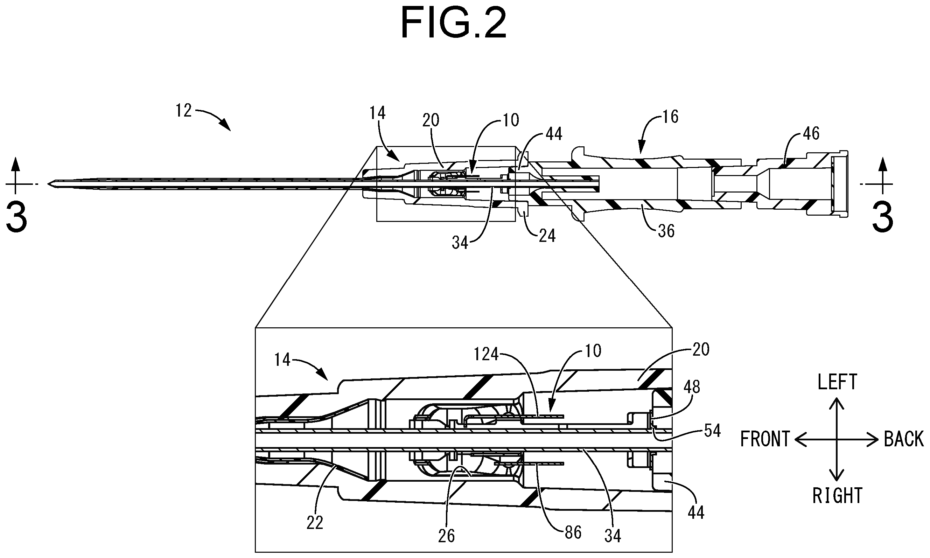

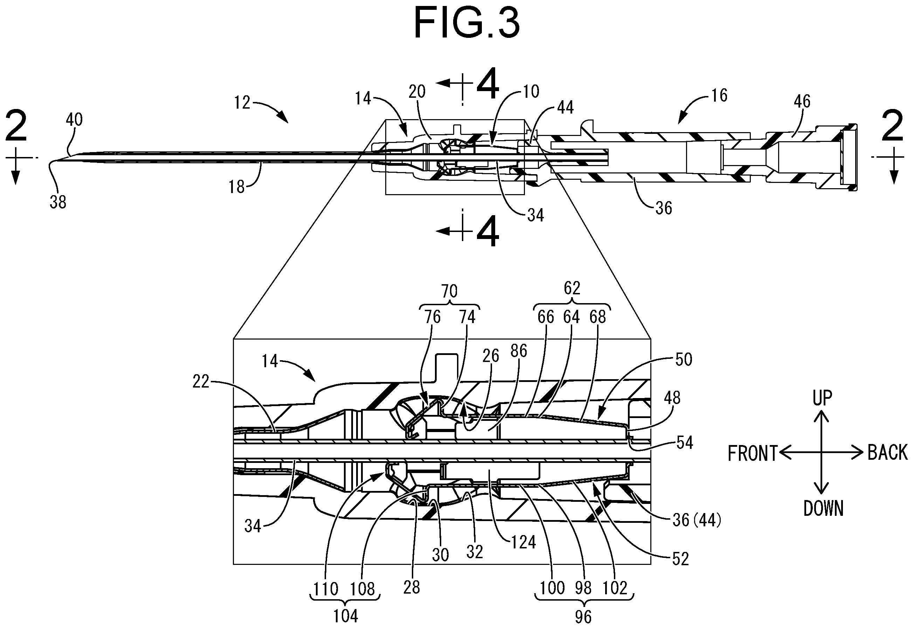

FIGS. 1 to 4 show an indwelling needle assembly 12 including a needle tip protector 10 according to a first practical embodiment of the present invention. The indwelling needle assembly 12 is constituted by an outer needle unit 14 and an inner needle unit 16 being combined. In the following description, as a general rule, the front-back direction refers to the left-right direction in FIG. 2, which is the needle axis direction. The vertical direction refers to the vertical direction in FIG. 3, and the left-right direction refers to the vertical direction in FIG. 2. Besides, as a general rule, the left side in FIG. 2, which is the side of a needle tip 38 (to be described later), is the distal end, and the right side in FIG. 2 is the proximal end.

The outer needle unit 14 has a structure in which an outer needle hub 20 is fixed to the proximal end portion of an outer needle 18. The outer needle 18 is a tube-shaped member made of synthetic resin or the like. The outer diameter dimension of the distal end portion of the outer needle 18 decreases toward the distal end. The proximal end portion of the outer needle 18 is inserted into the distal end portion of the tubular outer needle hub 20, and is fixed to the outer needle hub 20 by a caulking pin 22 that is fitted into the proximal end portion of the outer needle 18 and the distal end portion of the outer needle hub 20.

The outer needle hub 20 is formed of rigid synthetic resin, for example. The outer needle hub 20 is a member having an approximately round tubular shape overall. The outer needle hub 20 has a luer taper on its inner circumferential surface that increases in diameter toward the proximal end side. An external circuit such as a syringe or infusion line can be connected to the lumen of the outer needle hub 20 by fitting a syringe tip or a connector (not shown) to the lumen of the outer needle hub 20. In addition, a screw part 24 protruding radially outward is formed at the proximal end part of the outer needle hub 20. By the screw part 24 being screwed with a locking part (not shown), it is possible to prevent the external circuit from accidentally slipping out of the outer needle hub 20.

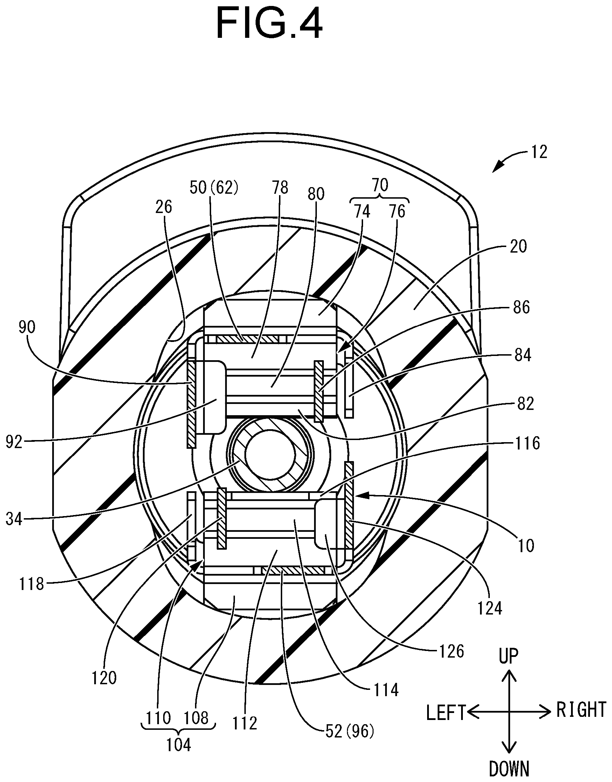

The lumen of the outer needle hub 20 includes a housing part 26. The housing part 26 is provided in the axially middle portion of the outer needle hub 20. The housing part 26 is provided by the lumen of the outer needle hub 20 being expanded in a concave shape to the vertically opposite sides. As shown in FIG. 4, the inner circumferential surface of the housing part 26 has an elliptical transverse cross section, and the diameter dimension in the vertical direction is larger than the diameter dimension in the left-right direction. The inner circumferential surface on each of the upper and lower sides of the housing part 26 has a concave shape that opens radially inward when viewed in vertical cross section shown in FIG. 3, and comprises a distal tapered inner circumferential surface 28, a straight inner circumferential surface 30, and a proximal tapered inner circumferential surface 32. The inner circumferential surface of the housing part 26 is not limited to the shape having an elliptical transverse cross section, but may be formed by being expanded in a concave shape about the entire circumference.

The distal tapered inner circumferential surface 28 inclines so as to expand radially outward in the vertical direction toward the proximal end side, and its inclination angle with respect to the front-back direction decreases from the distal end side toward the proximal end side. The straight inner circumferential surface 30 extends straightly in the front-back direction with an approximately constant diameter dimension. The proximal tapered inner circumferential surface 32 inclines so as to be constricted radially inward toward the proximal end side, and its inclination angle with respect to the front-back direction increases from the distal end side toward the proximal end side. Therefore, the vertical inside dimension of the housing part 26 gradually increases from the distal end side, reaches a maximum over a predetermined length in the front-back middle portion, and gradually decreases toward the proximal end side, so that the housing part 26 has the concave shape overall when viewed in vertical cross section in FIG. 3.

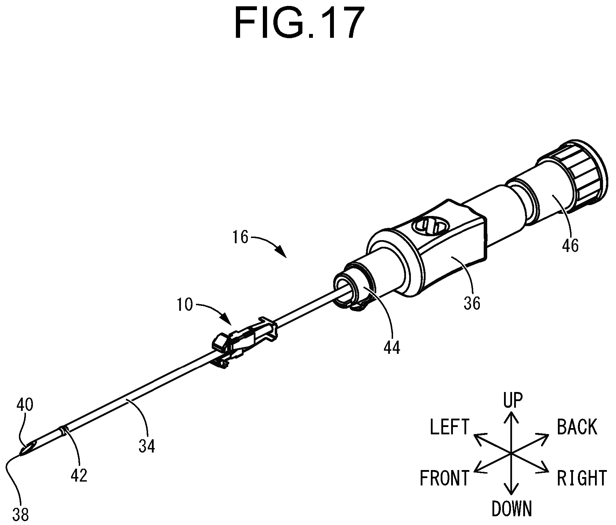

The inner needle unit 16 has a structure in which an inner needle hub 36 is attached to the proximal end portion of an inner needle 34 serving as a puncture needle. The inner needle 34 is a hollow needle formed of medical metal such as stainless steel, and has a sharp needle tip 38 at the distal end. The needle tip 38 is formed by the distal end portion of the inner needle 34 having a blade surface 40 that extends so as to incline with respect to the needle axis. Near the distal end of the inner needle 34, provided is a locking part 42 that is partially enlarged in diameter (see FIG. 17). The proximal end portion of the inner needle 34 is inserted into the distal end portion of the tubular inner needle hub 36, and those inner needle 34 and inner needle hub 36 are fixed by means of adhesion or the like.

The inner needle hub 36 is a member having an approximately round tubular shape, and is formed of rigid synthetic resin. The distal end part of the inner needle hub 36 includes a cylindrical connecting part 44 that can be fitted into the proximal end opening of the outer needle hub 20. A filter cap 46 is fitted into the proximal end opening of the inner needle hub 36.

The outer needle unit 14 and the inner needle unit 16 form the indwelling needle assembly 12 by being mutually combined in the state where the connecting part 44 of the inner needle hub 36 is fitted into the proximal end opening of the outer needle hub 20 and the inner needle 34 is inserted through the outer needle 18 and the outer needle hub 20. By applying a tensile force to the inner needle unit 16 toward the proximal end side with respect to the outer needle unit 14, the fitting of the connecting part 44 to the outer needle hub 20 will be released, so that the outer needle unit 14 and the inner needle unit 16 can be mutually separated.

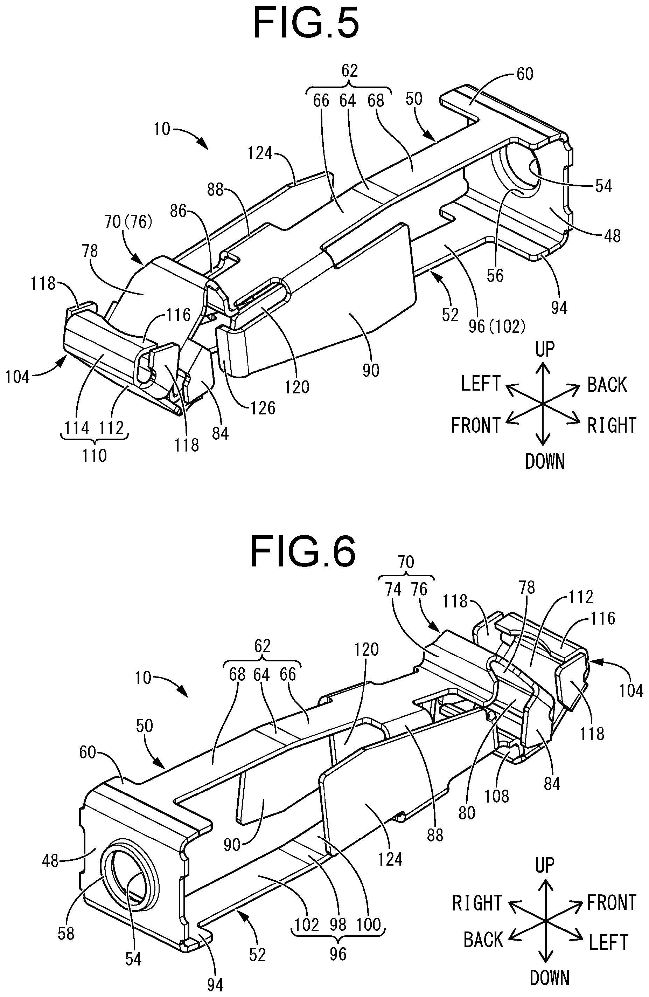

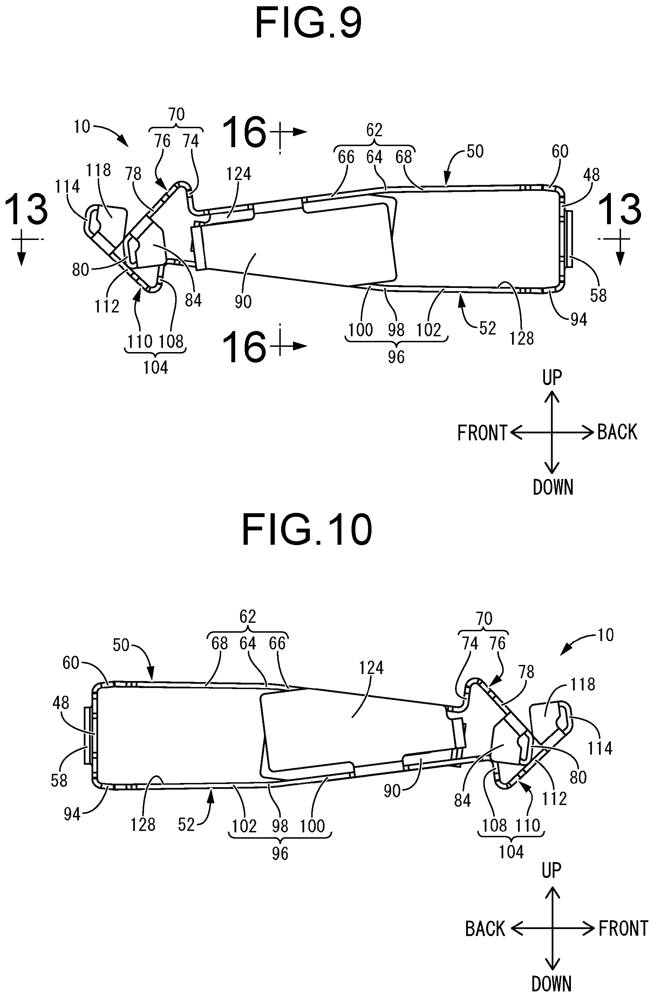

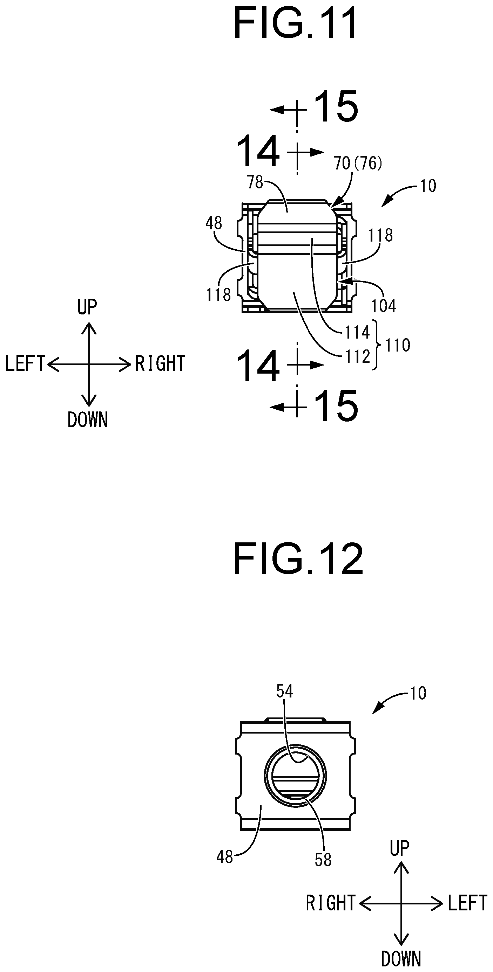

The needle tip protector 10 is attached to the inner needle 34. The needle tip protector 10 is externally attached to the inner needle 34, and moves to the distal end side of the inner needle 34 to prevent exposure of the needle tip 38 when the inner needle 34 is removed as described later. The needle tip protector 10 is made of medical metal such as stainless steel, for example. As shown in FIGS. 5 to 16, the needle tip protector 10 has a structure in which a first side plate 50 and a second side plate 52 extend out toward the needle tip 38 side from both edges of a bottom part 48 located on the needle base side (the opposite side of the needle tip 38) of the inner needle 34. In the present practical embodiment, the parts described later that constitute the needle tip protector 10 are integrally formed. Specifically, each part constituting the needle tip protector 10 is formed by bending the corresponding part of a single metal member.

As shown in FIGS. 5 and 6, the bottom part 48 is an approximately rectangular flat plate. At a facing pair of edges of the bottom part 48, the first side plate 50 and the second side plate 52 are integrally formed and extend out to the distal end side, which is one side in the plate thickness direction of the bottom part 48. In other words, the bottom part 48 integrally connects the proximal end parts of the first side plate 50 and the second side plate 52. The first side plate 50 and the second side plate 52 of the present practical embodiment are separated from and face each other, and do not extend in an intersecting manner.

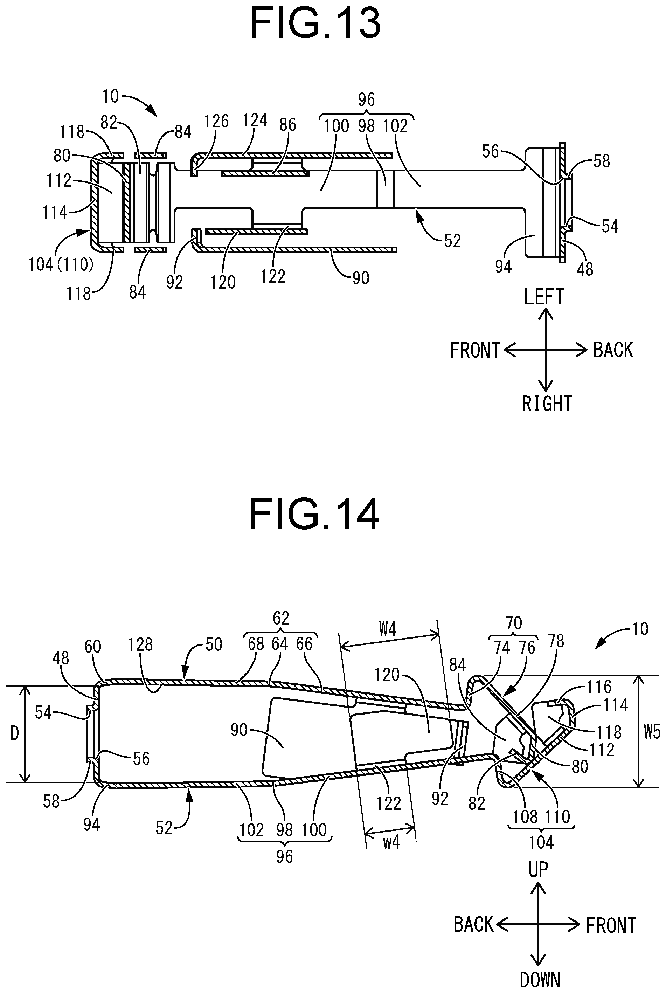

The bottom part 48 is penetrated by a needle insertion hole 54 in the plate thickness direction. The needle insertion hole 54 is a circular hole having such a size that allows the inner needle 34 to be inserted but does not allow the locking part 42 of the inner needle 34 to pass through. The needle insertion hole 54 is not limited in hole cross-sectional shape or the like as long as the inner needle 34 can be inserted. As shown in FIGS. 13 to 15, a needle guide surface 56 having an inclination angle is provided to the opening peripheral edge on the distal end side of the needle insertion hole 54 of the bottom part 48. The needle guide surface 56 is a curved surface with an arcuate cross section that expands such that the inclination angle with respect to the needle axis direction increases toward the distal end side. The radius of curvature of the needle guide surface 56 with the arcuate cross section is preferably within the range of 3% to 200% of the radius of the needle insertion hole 54, and more preferably within the range of 5% to 100% thereof. A reinforcing tube part 58 is provided to the opening peripheral edge on the proximal end side of the needle insertion hole 54 of the bottom part 48. As shown in FIG. 6, the reinforcing tube part 58 has an approximately round tubular shape provided along the opening peripheral edge of the needle insertion hole 54, and protrudes from the bottom part 48 to the proximal end side. The dimension of protrusion of the reinforcing tube part 58 from the bottom part 48 is preferably within the range of 3% to 200% of the diameter of the needle insertion hole 54, and more preferably within the range of 5% to 100% thereof. The needle guide surface 56 is not limited to a curved surface whose inclination angle varies from place to place, but may be, for example, a tapered surface extending at a constant inclination angle.

The first side plate 50 has a band plate shape extending in the front-back direction. The proximal end part of the first side plate 50, which is connected with the bottom part 48, comprises a first wide part 60 having approximately the same left-right width dimension as the bottom part 48. The first side plate 50 includes a first narrow part 62, which is smaller in left-right width dimension than the maximum width dimension of the needle-tip cover part, on the distal end side with respect to the first wide part 60. The first narrow part 62 is the portion excluding the connection portion with a first lateral cover piece 86 and a first protrusion piece 90 described later, and is a low rigidity part that can be easily flexed. The first narrow part 62 is preferably, but not exclusively, formed without ribs or the like extending in the length direction (approximately the front-back direction), and is formed with an approximately constant thickness dimension in its entirety. The first narrow part 62 is formed to be located in the middle portion in the left-right direction of the first side plate 50 (the first wide part 60) by its left and right ends being located closer to the center in the left-right direction than the left and right ends of the bottom part 48 (the first wide part 60). The left and right ends of the first narrow part 62 may be located closer to the center in the left-right direction from the left and right ends of the bottom part 48 by mutually the same left-right dimension. However, it is desirable that the central axis of the first narrow part 62 be shifted from the center in the left-right direction of the bottom part 48 for the purpose of facilitating the formation of the first lateral cover piece 86 at the appropriate location and the like. For example, the distance in the left-right direction between the left end (or the right end) of the first narrow part 62 and the left end (or the right end) of the bottom part 48 is 1.5 times or more as large as the distance in the right-right direction between the right end (or the left end) of the first narrow part 62 and the right end (or the left end) of the bottom part 48, and more preferably twice or more.

The first side plate 50 includes a first intermediate connecting part 64 in the midway in the length direction, and the distal end side with respect to the first intermediate connecting part 64 comprises a first distal end portion 66, while the proximal end side with respect to the first intermediate connecting part 64 comprises a first proximal end portion 68. Regarding the first side plate 50 in the isolated state of the needle tip protector 10, the inclination angle of the first distal end portion 66 with respect to the needle axis direction is greater than that of the first proximal end portion 68. In the present practical embodiment, in the isolated state of the needle tip protector 10, the first proximal end portion 68 is approximately parallel to the needle axis direction. Besides, in the isolated state of the needle tip protector 10, the first distal end portion 66 inclines to the second side plate 52 side toward the distal end side. The first intermediate connecting part 64 is preferably located within the range of one-fourth to three-fourths in the length direction of the first side plate 50, and in the present practical embodiment, the first intermediate connecting part 64 is located at approximately the center. The first intermediate connecting part 64 may be, for example, a curved part where the plate is bent, or may have a tapered surface with an inclination angle greater than that of the first proximal end portion 68 and smaller than that of the first distal end portion 66. Moreover, the first wide part 60 at the proximal end part of the first side plate 50 of the present practical embodiment is not essential.

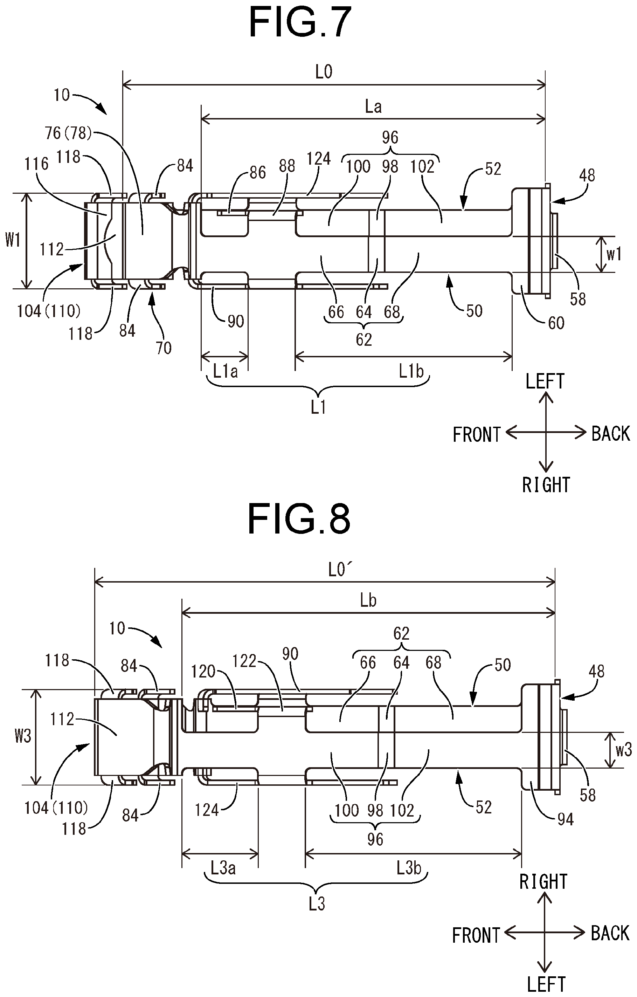

It is desirable that the length dimension L1 of the first narrow part 62 be obtained sufficiently long relative to the length dimension La of the first side plate 50. That is, the length dimension L1 of the first narrow part 62 is preferably within the range of 50% to 100% of the length dimension La of the first side plate 50, and more preferably within the range of 60% to 95% thereof. The length dimension L1 of the first narrow part 62 is preferably, for example, at least 30% of the entire length dimension L0 of the first side plate 50 and a first needle-tip cover part 70 combined, and more preferably at least 50% thereof. As shown in FIG. 7, the length dimension L1 of the first narrow part 62 is the sum of the length dimension L1a of the distal end side and the length dimension L1b of the proximal end side with respect to a first connection portion 88 (described later).

It is desirable that the first narrow part 62 be narrower in the left-right direction than the first needle-tip cover part 70 described later. The width dimension w1 of the first narrow part 62 is preferably within the range of 15% to 70% of the maximum width dimension W1 of the first needle-tip cover part 70, and more preferably within the range of 25% to 50% thereof. This reduces the bending deformation rigidity of the first side plate 50 in the plate thickness direction. As shown in FIG. 7, the dimension W1 is the width dimension including a pair of first lateral guarding pieces 84, 84.

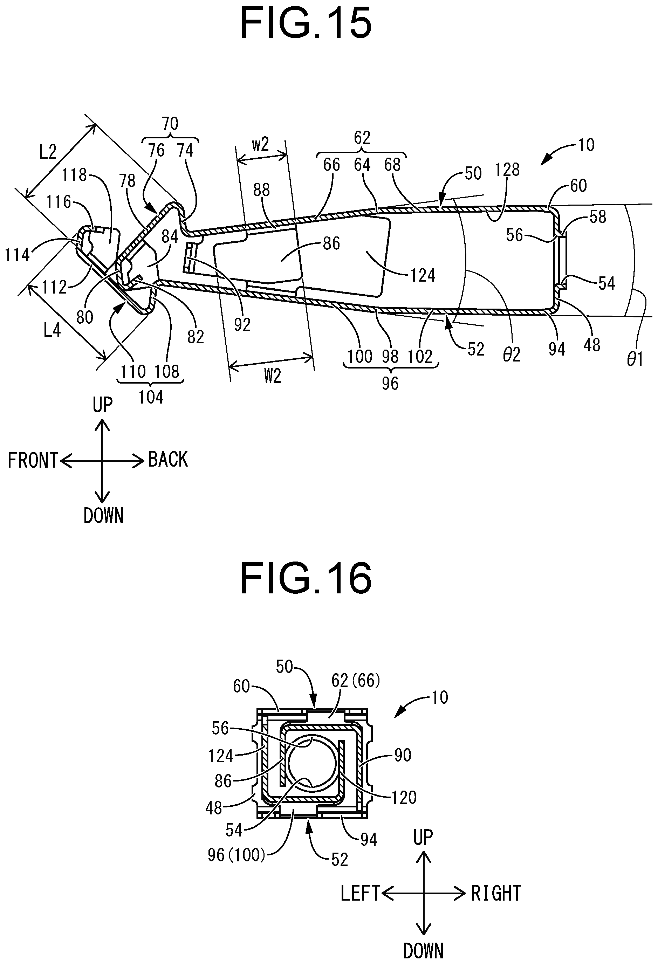

The first needle-tip cover part 70 is provided on the distal end side of the first side plate 50. The first needle-tip cover part 70 includes a first stepped part 74 protruding from the distal end of the first side plate 50 to the opposite side of the second side plate 52, and a first needle-tip protection part 76 protruding from the protruding distal end of the first stepped part 74 toward the distal end side of the second side plate 52. The first needle-tip protection part 76 protrudes from the first side plate 50 toward the distal end side, while protruding toward the second side plate 52 side. The first needle-tip protection part 76 comprises a first distal-end protection piece 78 provided continuous with the first stepped part 74, and a first return piece 80 protruding from the distal end of the first distal-end protection piece 78 toward the second side plate 52 side. The first distal-end protection piece 78 has an approximately rectangular flat plate shape that extends obliquely toward the distal end side while inclining toward the second side plate 52 side. Regarding the first return piece 80, which is continuous with the distal end side of the first distal-end protection piece 78, the angle formed with the first distal-end protection piece 78 is an obtuse angle that is greater than 90° in the present practical embodiment. The length dimension L2 of the first needle-tip cover part 70 is preferably within the range of 50% to 150% of the maximum width dimension W1 of the first needle-tip cover part 70, and more preferably within the range of 70% to 130% thereof. As shown in FIG. 15, the dimension L2 is the length dimension of the first needle-tip cover part 70 in the direction parallel to the first distal-end protection piece 78.

A first needle-tip stopper part 82 is provided at the protruding distal end of the first return piece 80. The first needle-tip stopper part 82 has an approximately rectangular plate shape. The first needle-tip stopper part 82 protrudes from the first return piece 80 toward the proximal end side. The first needle-tip stopper part 82 of the present practical embodiment extends approximately parallel to the first distal-end protection piece 78. The first needle-tip stopper part 82 is formed by bending and is integrally continuous with the first return piece 80.

The pair of first lateral guarding pieces 84, 84 are provided on the left and right opposite sides of the distal end portion of the first distal-end protection piece 78. Each first lateral guarding piece 84 has a plate shape spreading approximately orthogonally to the left-right direction. The first lateral guarding piece 84 protrudes from the first distal-end protection piece 78 toward the second side plate 52 side. The first lateral guarding piece 84 has a length that reaches the first needle-tip stopper part 82, thereby making it difficult for the needle tip 38 to be exposed to the lateral side in the needle-tip protection state described later.

The first side plate 50 includes the first lateral cover piece 86. As shown in FIGS. 15 and 16, the first lateral cover piece 86 protrudes vertically inward (downward) from one edge in the left-right direction of the first side plate 50 toward the second side plate 52. The first connection portion 88, which is a connection portion with the first lateral cover piece 86, is provided at the one edge of the first side plate 50 in the left-right direction. The first lateral cover piece 86 is located in the middle of the first side plate 50 in the length direction. The first lateral cover piece 86 is provided at the first distal end portion 66, which is the distal end side with respect to the first intermediate connecting part 64. Regarding the first lateral cover piece 86, the vertical dimension is approximately constant at the proximal end portion near the bottom part 48, while gradually decreasing toward the distal end at the distal end portion near the first needle-tip cover part 70.

The connection width dimension w2 of the first connection portion 88 in the front-back direction is smaller than the maximum width dimension W2 of the first lateral cover piece 86 in the same direction. The connection width dimension w2 of the first connection portion 88 is preferably within the range of 20% to 80% of the maximum width dimension W2 of the first lateral cover piece 86 in the same direction, and more preferably within the range of 30% to 70% thereof. Besides, the connection width dimension w2 of the first connection portion 88 is preferably within the range of 5% to 30% of the length dimension La of the first side plate 50, and more preferably within the range of 10% to 20% thereof. Accordingly, the first lateral cover piece 86 is stably supported by the first side plate 50, and the length of the first narrow part 62 provided off the first connection portion 88 is sufficiently obtained, thereby suppressing the deformation rigidity of the first side plate 50. As shown in FIG. 15, the dimensions w2 and W2 are the dimensions in the direction parallel to the first distal end portion 66, which is inclined with respect to the front-back direction in the isolated state of the needle tip protector 10.

The first side plate 50 includes a first protrusion piece 90. As shown in FIG. 16, the first protrusion piece 90 protrudes vertically inward (downward) from the other edge in the left-right direction of the first side plate 50 toward the second side plate 52. The first protrusion piece 90 is located in the middle of the first side plate 50 in the length direction, and is provided at the first distal end portion 66, which is the distal end side with respect to the first intermediate connecting part 64. The first protrusion piece 90 is provided at the edge of the first side plate 50 opposite to the first lateral cover piece 86 in the left-right direction. The first protrusion piece 90 is separated from and face the first lateral cover piece 86 in the left-right direction. In the present practical embodiment, the first lateral cover piece 86 and the first protrusion piece 90 spread approximately parallel to each other. As shown in FIG. 13, the first protrusion piece 90 has a length dimension larger than that of the first lateral cover piece 86. The first protrusion piece 90 extends to the opposite outer sides with respect to the first lateral cover piece 86 in the front-back length direction. Like the first lateral cover piece 86, regarding the first protrusion piece 90, the vertical dimension is approximately constant at the proximal end portion near the bottom part 48, while decreasing toward the distal end at the distal end portion near the first needle-tip cover part 70. As shown in FIG. 13, at the end (the front end) of the first protrusion piece 90 on the first needle-tip cover part 70 side, a first regulating piece 92 is integrally formed so as to protrude inward in the left-right direction and regulate displacement of the inner needle 34 to the left and right lateral sides. The connection width dimension of the connection portion between the first side plate 50 and the first protrusion piece 90 is approximately the same as the connection width dimension w2 of the first connection portion 88, which is the connection portion between the first side plate 50 and the first lateral cover piece 86. The connection width dimension of the connection portion between the first side plate 50 and the first protrusion piece 90 is preferably not greater than 70% of the length dimension of the first protrusion piece 90 in the direction parallel to the said connection portion, and is preferably not greater than 50% thereof in particular.

The second side plate 52 has a band plate shape extending in the front-back direction. The proximal end part of the second side plate 52, which is connected with the bottom part 48, comprises a second wide part 94 having approximately the same left-right width dimension as the bottom part 48. In the second side plate 52, the distal end side with respect to the second wide part 94, which excludes the connection portion with a second lateral cover piece 120 and a second protrusion piece 124 described later, comprises a second narrow part 96 whose left-right width dimension is smaller than the maximum width dimension of the needle-tip cover part. The second narrow part 96 is a low rigidity part that can be easily deformed. The second narrow part 96 is preferably, but not exclusively, provided with no ribs or the like extending in the length direction, which increases the deformation rigidity, and has an approximately constant thickness dimension in its entirety. The second narrow part 96 is formed to be located in the middle portion in the left-right direction of the second side plate 52 (the second wide part 94) by its left and right ends being located closer to the center in the left-right direction than the left and right ends of the bottom part 48 (the second wide part 94). The left and right ends of the second narrow part 96 may be located closer to the center in the left-right direction from the left and right ends of the bottom part 48 (the second wide part 94) by mutually the same left-right dimension. However, it is desirable that the central axis of the second narrow part 96 be shifted from the center in the left-right direction of the bottom part 48 for the purposes of facilitating the formation of the second lateral cover piece 120 at the appropriate location and the like. For example, the distance in the left-right direction between the left end (or the right end) of the second narrow part 96 and the left end (or the right end) of the bottom part 48 is 1.5 times or more as large as the distance in the right-right direction between the right end (or the left end) of the second narrow part 96 and the right end (or the left end) of the bottom part 48, and more preferably twice or more.

The second side plate 52 includes a second intermediate connecting part 98 in the midway in the length direction, and the distal end side with respect to the second intermediate connecting part 98 comprises a second distal end portion 100, while the proximal end side with respect to the second intermediate connecting part 98 comprises a second proximal end portion 102. Regarding the second side plate 52 in the isolated state of the needle tip protector 10, the inclination angle of the second distal end portion 100 with respect to the needle axis direction is greater than that of the second proximal end portion 102. In the present practical embodiment, in the isolated state of the needle tip protector 10, the second proximal end portion 102 is approximately parallel to the needle axis direction. Besides, in the isolated state of the needle tip protector 10, the second distal end portion 100 inclines to the first side plate 50 side toward the distal end side. The second intermediate connecting part 98 is preferably located 1/4 to 3/4 of the length dimension of the second side plate 52 away from the proximal end, and in the present practical embodiment, the second intermediate connecting part 98 is located approximately in the center of the length direction of the second side plate 52. The second intermediate connecting part 98 may be, for example, a curved part where the plate is bent, or may have a tapered surface with an inclination angle greater than that of the second proximal end portion 102 and smaller than that of the second distal end portion 100.

It is desirable that the length dimension L3 of the second narrow part 96 be obtained sufficiently long relative to the length dimension Lb of the second side plate 52. That is, the length dimension L3 of the second narrow part 96 is preferably within the range of 50% to 100% of the length dimension Lb of the second side plate 52, and more preferably within the range of 60% to 95% thereof. The length dimension L3 of the second narrow part 96 is preferably, for example, at least 30% of the entire length dimension L0' of the second side plate 52 and a second needle-tip cover part 104 (described later) combined, and more preferably at least 50% thereof. As shown in FIG. 8, the length dimension L3 of the second narrow part 96 is the sum of the length dimension L3a of the distal end side and the length dimension L3b of the proximal end side with respect to a second connection portion 122. As shown in FIGS. 7 and 8, the dimensions L0, L0', L1, and L3 are length dimensions in the front-back direction. Each of the said length dimensions is understood as a length in the front-back direction (the front-back length as viewed in the vertical direction), even if, for example, the first and second side plates 50, 52 are inclined partially or entirely with respect to the front-back direction.

It is desirable that the second narrow part 96 be narrower in the left-right direction than the second needle-tip cover part 104 described later. The width dimension w3 of the second narrow part 96 is preferably within the range of 15% to 70% of the maximum width dimension W3 of the second needle-tip cover part 104, and more preferably within the range of 25% to 50% thereof. This reduces the bending deformation rigidity of the second side plate 52 in the plate thickness direction. As shown in FIG. 8, the dimension W3 is the width dimension including a pair of second lateral guarding pieces 118, 118.

The second needle-tip cover part 104 is provided at the distal end side of the second side plate 52. The second needle-tip cover part 104 includes a second stepped part 108 protruding from the distal end of the second side plate 52 to the opposite side of the first side plate 50, and a second needle-tip protection part 110 protruding from the protruding distal end of the second stepped part 108 toward the distal end side of the first side plate 50. It is desirable that the second stepped part 108, which is the proximal end part of the second needle-tip cover part 104, be located at a different position in the front-back direction from the first stepped part 74, which is the proximal end part of the first needle-tip cover part 70. In the present practical embodiment, the first stepped part 74 is located on the proximal end side with respect to the second stepped part 108. In the present practical embodiment, the length of extension of the first needle-tip protection part 76 forward from the first stepped part 74 is approximately the same as the length of extension of the second needle-tip protection part 110 forward from the second stepped part 108. With this configuration, due to the shift of the first stepped part 74 and the second stepped part 108 in the front-back direction, the distal end (the front end) of the first needle-tip protection part 76 is located on the proximal end side (the back side) with respect to the distal end part of the second needle-tip protection part 110.

The second needle-tip protection part 110 protrudes from the second side plate 52 toward the distal end side, while protruding toward the first side plate 50. The second needle-tip protection part 110 comprises a second distal-end protection piece 112 provided continuous with the second stepped part 108, and a second return piece 114 protruding from the distal end of the second distal-end protection piece 112 toward the first side plate 50 side. The second distal-end protection piece 112 has an approximately rectangular flat plate shape that extends obliquely toward the distal end side while inclining toward the first side plate 50 side. Regarding the second return piece 114, which is continuous with the distal end side of the second distal-end protection piece 112, the angle formed with the second distal-end protection piece 112 is an obtuse angle that is greater than 90° in the present practical embodiment. The length dimension L4 of the second needle-tip cover part 104 is preferably within the range of 50% to 150% of the maximum width dimension W3 of the second needle-tip cover part 104, and more preferably within the range of 70% to 130% thereof. As shown in FIG. 15, the dimension L4 is the length dimension of the second needle-tip cover part 104 in the direction parallel to the second distal-end protection piece 112.

A second needle-tip stopper part 116 is provided at the protruding distal end of the second return piece 114. The second needle-tip stopper part 116 has an approximately rectangular plate shape. The second needle-tip stopper part 116 protrudes from the second return piece 114 toward the proximal end side. The second needle-tip stopper part 116 of the practical embodiment extends approximately orthogonally to the second return piece 114. The second needle-tip stopper part 116 is formed by bending and is integrally continuous with the second return piece 114.

The pair of second lateral guarding pieces 118, 118 are provided on the left and right opposite sides of the distal end portion of the second distal-end protection piece 112. Each second lateral guarding piece 118 has a plate shape spreading approximately orthogonally to the left-right direction. The second lateral guarding piece 118 protrudes from the second distal-end protection piece 112 toward the first side plate 50 side. The second lateral guarding piece 118 has a length that reaches the second needle-tip stopper part 116.

The second side plate 52 includes a second lateral cover piece 120. As shown in FIGS. 14 and 16, the second lateral cover piece 120 protrudes vertically inward (upward) from the other edge in the left-right direction of the second side plate 52 toward the first side plate 50. The second connection portion 122, which is a connection portion with the second lateral cover piece 120, is provided at the other edge in the left-right direction of the second side plate 52. The second lateral cover piece 120 is located in the middle of the second side plate 52 in the length direction. The second lateral cover piece 120 is provided at the second distal end portion 100, which is the distal end side with respect to the second intermediate connecting part 98. Regarding the second lateral cover piece 120, the vertical dimension is approximately constant at the proximal end portion near the bottom part 48, while gradually decreasing toward the distal end at the distal end portion near the second needle-tip cover part 104.

The connection width dimension w4 of the second connection portion 122 in the front-back direction is smaller than the maximum width dimension W4 of the second lateral cover piece 120 in the same direction. The connection width dimension w4 of the second connection portion 122 is preferably within the range of 20% to 80% of the maximum width dimension W4 of the second lateral cover piece 120 in the same direction, and more preferably within the range of 30% to 70% thereof. Besides, the connection width dimension w4 of the second connection portion 122 is preferably within the range of 5% to 30% of the length dimension Lb of the second side plate 52, and more preferably within the range of 10% to 20% thereof. By setting the connection width dimension w4 of the second connection portion 122 in this way, the second lateral cover piece 120 is stably supported by the second side plate 52, and the length of the second narrow part 96 provided off the second connection portion 122 is sufficiently obtained, thereby suppressing the deformation rigidity of the second side plate 52. As shown in FIG. 14, the dimensions w4 and W4 are the dimensions in the direction parallel to the second distal end portion 100, which is inclined with respect to the front-back direction in the isolated state of the needle tip protector 10.

The second side plate 52 includes a second protrusion piece 124. As shown in FIGS. 15 and 16, the second protrusion piece 124 extends from the one edge in the left-right direction of the second side plate 52 toward the first side plate 50. The second protrusion piece 124 is located in the middle of the second side plate 52 in the length direction, and is provided at the second distal end portion 100, which is the distal end side with respect to the second intermediate connecting part 98. The second protrusion piece 124 is provided at the edge of the second side plate 52 opposite to the second lateral cover piece 120 in the left-right direction. The second protrusion piece 124 is separated from and face the second lateral cover piece 120 in the left-right direction. In the present practical embodiment, the second lateral cover piece 120 and the second protrusion piece 124 spread approximately parallel to each other. As shown in FIG. 13, the second protrusion piece 124 has a length dimension larger than that of the second lateral cover piece 120. The second protrusion piece 124 extends to the opposite outer sides with respect to the second lateral cover piece 120 in the length direction. Like the second lateral cover piece 120, regarding the second protrusion piece 124, the vertical dimension is approximately constant at the proximal end portion near the bottom part 48, while decreasing toward the distal end at the distal end portion near the second needle-tip cover part 104. As shown in FIG. 13, at the end (the front end) of the second protrusion piece 124 on the second needle-tip cover part 104 side, a second regulating piece 126 is integrally formed so as to protrude inward in the left-right direction and regulate displacement of the inner needle 34 to the left and right lateral sides. The connection width dimension of the connection portion between the second side plate 52 and the second protrusion piece 124 is approximately the same as the connection width dimension w4 of the second connection portion 122, which is the connection portion between the second side plate 52 and the second lateral cover piece 120. The connection width dimension of the connection portion between the second side plate 52 and the second protrusion piece 124 is preferably not greater than 70% of the length dimension of the second protrusion piece 124 in the direction parallel to the said connection portion, and is preferably not greater than 50% thereof in particular.

The first lateral cover piece 86 and the second protrusion piece 124 are provided at the same side edge in the left-right direction in the first side plate 50 and the second side plate 52, and overlap each other in the left-right direction in the state of attachment to the inner needle 34 before the needle tip is protected. As shown in FIG. 16, the first lateral cover piece 86 is positioned inwardly apart from the second protrusion piece 124 in the left-right direction. The first lateral cover piece 86 has a protrusion height that does not reach the second side plate 52. The second protrusion piece 124 has a protrusion height that does not reach the first side plate 50. The first lateral cover piece 86 and the second protrusion piece 124 partially overlap each other. As shown in FIG. 15, the second protrusion piece 124 extends to the opposite outer sides with respect to the first lateral cover piece 86 in the length direction.

As shown in FIG. 16, the second lateral cover piece 120 and the first protrusion piece 90 are provided at the same side edge in the left-right direction in the first side plate 50 and the second side plate 52, and overlap each other in the left-right direction in the state of attachment to the inner needle 34 before the needle tip is protected. The second lateral cover piece 120 is positioned inwardly apart from the first protrusion piece 90 in the left-right direction. The second lateral cover piece 120 has a protrusion height that does not reach the first side plate 50. The first protrusion piece 90 has a protrusion height that does not reach the second side plate 52. The second lateral cover piece 120 and the first protrusion piece 90 partially overlap each other. As shown in FIG. 14, the first protrusion piece 90 extends to the opposite outer sides with respect to the second lateral cover piece 120 in the front-back length direction. In this way, the length dimensions of the first and second protrusion pieces 90, 124 in the front-back direction are set long, thereby making it more difficult for the needle tip 38 to be exposed to the left and right lateral sides in the needle-tip protection state described later.

The overlap between the first and second lateral cover pieces 86, 120 and the first and second protrusion pieces 90, 124 in this way prevents external exposure of the inner needle 34 through the gap between the first lateral cover piece 86 and the second side plate 52 and the gap between the second lateral cover piece 120 and the first side plate 50. Besides, for example, even if the first and second side plates 50, 52 undergo flexural deformation in the direction of mutual separation, the overlap between the first and second lateral cover pieces 86, 120 and the first and second protrusion pieces 90, 124 is maintained without being released, thereby preventing exposure of the inner needle 34 to the left and right lateral sides.

As shown in FIG. 16, the first lateral cover piece 86 and the second lateral cover piece 120 are provided on the opposite sides in the left-right direction, and are separated from and face each other in the left-right direction. Besides, the first protrusion piece 90 and the second protrusion piece 124 are provided on the opposite sides in the left-right direction, and are separated from and face each other in the left-right direction. The first protrusion piece 90 and the second protrusion piece 124 are located on the opposite outer sides in the left-right direction with respect to the first and second lateral cover pieces 86, 120.

As shown in FIGS. 14 and 15, the first and second lateral cover pieces 86, 120 and the first and second protrusion pieces 90, 124 are all separated from the bottom part 48 to the distal end side in the length direction. A molding space 128 is formed between the bottom part 48 and the first and second lateral cover pieces 86, 120 as well as the first and second protrusion pieces 90, 124, the molding space 128 penetrating in the left-right direction and being opened to the opposite sides in the left-right direction between the facing surfaces of the first and second side plates 50, 52. In the present practical embodiment, the first and second protrusion pieces 90, 124 extend out to near the first and second intermediate connecting parts 64, 98, and the molding space 128 is provided on the proximal end side with respect to the first and second intermediate connecting parts 64, 98. In the present practical embodiment, the first and second protrusion pieces 90, 124 protrude to the proximal end side with respect to the first and second lateral cover pieces 86, 120, so that the size of the molding space 128 is limited by the first and second protrusion pieces 90, 124. Therefore, exposure of the inner needle 34 to the outside through the molding space 128 is reduced, and troubles such as other needle tip protectors 10 entering into the molding space 128 and becoming entangled are avoided when a number of needle tip protectors 10 are housed and stored in the container, for example.

The needle tip protector 10 is formed, for example, by bending a blank metal plate punched into a predetermined shape by a press working (progressive press). During the press working, it is possible to process while holding down the bottom part 48 by utilizing the molding space 128. That is, during the pressing working, the portion corresponding to the bottom part 48 is clasped in the plate thickness direction by a support jig (not shown), which supports one surface, and a pressing jig (not shown), which supports the other surface. The pressing jig is shaped to be able to be inserted through the molding space 128 and is overlapped with the bottom part 48 on the side from which the first and second side plates 50, 52 rise up. This makes it possible to hold the bottom part 48 in a clasped state from the start to the end of the press working, thereby facilitating molding of the needle tip protector 10. The pressing jig also functions as a die for bending and forming the first and second side plates 50, 52 with respect to the bottom part 48. The blank metal plate to be processed into the needle tip protector 10 by the progressive press is formed, for example, by stainless steel or titanium for medical use, and is suitably a plate material having a thickness of 0.05 to 0.3 mm. By employing a blank metal plate in such a thickness range, it is possible to obtain a needle tip protector 10 with sufficiently low sliding resistance with respect to the inner needle 34 and with deformation rigidity that can stably maintain the needle tip protection mode, and damage or the like during the press working is also unlikely to be a problem. The needle tip protector 10 may have an approximately constant thickness dimension in its entirety, or may partially have thick-walled and/or thin-walled portions.

In the isolated state of the needle tip protector 10, the relative inclination angle θ1 between the first proximal end portion 68 of the first side plate 50 and the second proximal end portion 102 of the second side plate 52 is smaller than the relative inclination angle θ2 between the first distal end portion 66 and the second distal end portion 100. The size of the relative inclination angle θ1 between the first proximal end portion 68 and the second proximal end portion 102 is preferably within the range of -10° to 10°. In the present practical embodiment, the first proximal end portion 68 and the second proximal end portion 102 are approximately parallel to each other, and the angle θ1 is set to be approximately 0°. The size of the relative inclination angle θ2 between the first distal end portion 66 and the second proximal end portion 102 is preferably within the range of 5° to 30°. For example, it would also be acceptable that the first proximal end portion 68 and the second proximal end portion 102 each incline vertically outward toward the distal end side, while the first distal end portion 66 and the second distal end portion 100 each incline vertically inward toward the distal end side.

In this way, the relative inclination angle θ1 between the first proximal end portion 68 and the second proximal end portion 102 is smaller than the relative inclination angle θ2 between the first distal end portion 66 and the second distal end portion 100, thereby improving the ease of attachment of the needle tip protector 10 to the outer needle hub 20 and downsizing the needle tip protector 10. Specifically, it is assumed that an engaging part protrudes from the inner circumferential surface, as in the proximal end side of the housing part 26 of the outer needle hub 20, for example. Here, in a structure where the first and second side plates are extended to the distal end at a constant angle without increasing the width of the bottom part 48 (the distance between the facing first and second side plates at the proximal end), for example, when attaching the needle tip protector to the outer needle hub 20, the first and second side plates, which were flexed by the stress caused by the needle-tip cover part (the stepped part) of the needle tip protector climbing over the engaging part of the outer needle hub 20, may come into contact with the inner needle 34, posing a risk of reducing the ease of attachment of the needle tip protector to the outer needle hub 20. In particular, when the inner needle 34 is thick, interference between the inner needle 34 and the first and second side plates of the needle tip protector tends to be a problem. On the other hand, in the case where the width of the bottom part 48 is increased and the first and second side plates are extended to the distal end at a constant angle, the needle tip protector becomes larger. Therefore, in the present practical embodiment, in the isolated state of the needle tip protector 10 that is not attached to the inner needle 34, the relative inclination angle θ1 between the first and second proximal end portions 68, 102 is smaller than the relative inclination angle θ2 between the first and second distal end portions 66, 100. This suppresses both reduction in the ease of attachment of the needle tip protector 10 to the outer needle hub 20 and increase in the size of the needle tip protector 10 in the state of being attached to the inner needle 34. Besides, in the isolated state of the needle tip protector 10 that is not attached to the inner needle 34, it is easy to set the distance between the facing surfaces of the first and second side plates 50, 52 in their initial shape where no external force is exerted to such a size that prevents entry of the first and second needle-tip cover parts 70, 104 in their initial shape where no external force is exerted.

The first intermediate connecting part 64 and the second intermediate connecting part 98 are formed by bending each intermediate portion of the first narrow part 62 and the second narrow part 96 during the forming of the needle tip protector 10 by press working. In bending the said first narrow part 62 and second narrow part 96, the relative inclination angle between the first distal end portion 66 and the first proximal end portion 68, and the relative inclination angle between the second distal end portion 100 and the second proximal end portion 102 are each set to an appropriate angle by a die (a jig). This allows, for example, an excellent protection structure of the needle tip 38 due to reliably obtaining the effective length of the arm and the overlap between the first and second needle-tip cover parts 70, 104 when protecting the needle tip while reducing the maximum radial dimension.

In the isolated state of the needle tip protector 10, the vertical distance D between the facing surfaces of the first side plate 50 and the second side plate 52 is set to such a size that prevents entry of the distal end portion of the needle tip protector 10 constituted by the first and second needle-tip cover parts 70, 104 in its initial shape without deformation by an external force. In the present practical embodiment, in the isolated state of the needle tip protector 10, the vertical distance D between the facing surfaces of the first side plate 50 and the second side plate 52 is smaller than at least one of the maximum width dimension W1 of the first needle-tip cover part 70 and the maximum width dimension W3 of the second needle-tip cover part 104. The maximum width dimension W1 of the first needle-tip cover part 70 and the maximum width dimension W3 of the second needle-tip cover part 104 have approximately the same size as each other, and the vertical distance D between the facing surfaces of the first side plate 50 and the second side plate 52 is smaller than both the maximum width dimension W1 of the first needle-tip cover part 70 and the maximum width dimension W3 of the second needle-tip cover part 104. With this configuration, for example, in the case where a plurality of needle tip protectors 10 in the isolated state are housed in a box, bottle, etc. for storage, transportation, and the like, the distal end portion of the needle tip protector 10, which is tapered in the vertical direction, is unlikely to enter the space between the vertically facing surfaces of the first side plate 50 and the second side plate 52 (the molding space 128) of another needle tip protector 10. Therefore, the plurality of needle tip protectors 10 are unlikely to become entangled with one another, thereby reducing the labor required to untangle them. The distance D between the facing surfaces of the first and second side plates 50, 52 is approximately constant at the facing portions of the first proximal end portion 68 and the second proximal end portion 102 in the present practical embodiment, while decreasing at an approximately constant rate toward the distal end side at the facing portions of the first distal end portion 66 and the second distal end portion 100. However, for example, the distance D may vary at the facing portions of the first proximal end portion 68 and the second proximal end portion 102, and the rate of change does not have to be constant at the facing portions of the first distal end portion 66 and the second distal end portion 100. Besides, the first and second side plates 50, 52 may partially have a widened part that is larger than the maximum width dimension W1 of the first needle-tip cover part 70 and the maximum width dimension W3 of the second needle-tip cover part 104. Even in this case, by adjusting the length of the widened part, it is possible to prevent the distal end portion of the needle tip protector 10, which is constituted by the first and second needle-tip cover parts 70, 104, from entering between the facing surfaces of the first and second side plates 50, 52 in the initial shape of the needle tip protector 10 without deformation by the external force. It would also be acceptable to provide a convex part that protrudes from the facing inner surfaces of the first and second side plates 50, 52 to partially shorten the distance D therebetween, so that the needle-tip cover parts 70, 104 do not enter the gap between the first and second side plates 50, 52 by abutment against the said convex part.

The vertical distance D between the facing surfaces of the first side plate 50 and the second side plate 52 is smaller than the maximum outside dimension W5 of the first needle-tip cover part 70 and the second needle-tip cover part 104 in the vertical direction. As shown in FIG. 14, in the present practical embodiment, the maximum outside dimension W5 of the first needle-tip cover part 70 and the second needle-tip cover part 104 in the vertical direction refers to the vertical distance between the upper end of the first needle-tip protection part 76 (the protruding distal end of the first stepped part 74) and the lower end of the second needle-tip protection part 110 (the protruding distal end of the second stepped part 108). This makes it possible to more effectively prevent the needle tip protectors 10 from getting entangled with one another due to the distal end portion of one needle tip protector 10 entering the molding space 128 of another needle tip protector 10.

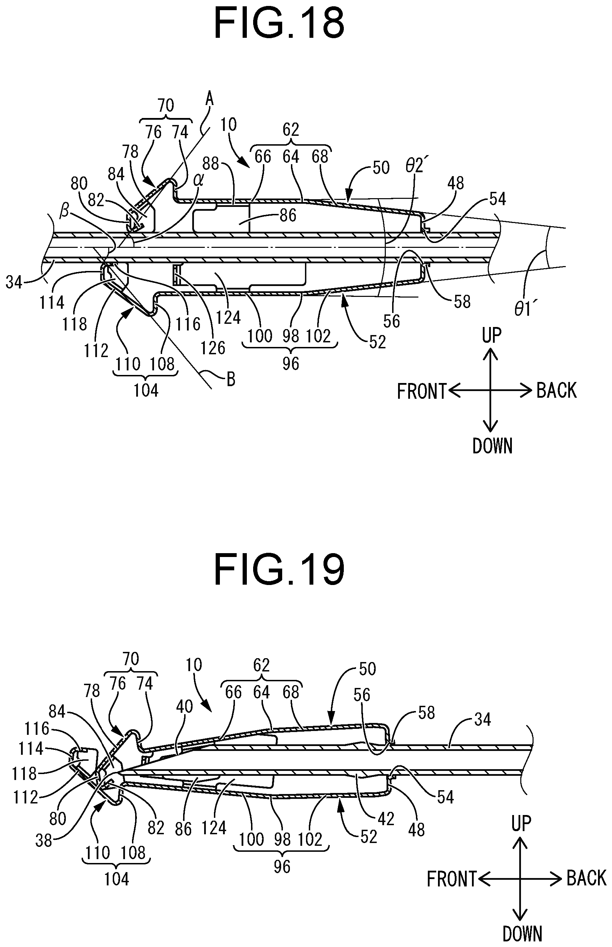

The needle tip protector 10 of the above construction is attached to the inner needle 34, as shown in FIGS. 17 and 18. That is, the inner needle 34 is inserted through the needle insertion hole 54 formed in the bottom part 48 of the needle tip protector 10, while being inserted between the first needle-tip cover part 70 and the second needle-tip cover part 104. With this configuration, the inner needle 34 is arranged to penetrate between the facing surfaces of the first side plate 50 and the second side plate 52 of the needle tip protector 10, and the needle tip protector 10 is externally attached onto the inner needle 34. The first side plate 50 and the second side plate 52 extend along the inner needle 34 on the opposite sides of the inner needle 34, and do not intersect in cross sectional view of the first side plate 50 and the second side plate 52. That is, when viewed in vertical cross section of the needle tip protector 10 attached to the inner needle 34 as shown in FIG. 18, the first side plate 50 and the second side plate 52 extend without intersecting the inner needle 34, and the distal end and the proximal end of the first side plate 50 are both located on the same side (the upper side) of the inner needle 34, while the distal end and the proximal end of the second side plate 52 are both located on the same side (the lower side) of the inner needle 34. The inner needle 34 is located between the facing surfaces of the first lateral cover piece 86 and the second lateral cover piece 120 of the needle tip protector 10, and is surrounded in part in the needle axis direction by the first and second side plates 50, 52, the first and second lateral cover pieces 86, 120, and the first and second protrusion pieces 90, 124. In FIG. 17, for illustrative purposes, the needle tip protector 10 is positioned away from the inner needle hub 36 to the needle tip 38 side. However, as shown in FIGS. 2 and 3, the needle tip protector 10 is positioned with the bottom part 48 in contact with or close to the distal end of the inner needle hub 36.

After the needle tip protector 10 is attached to the inner needle 34 in an isolated state without the inner needle hub 36, for example, the proximal end part of the inner needle 34 is fixed to the inner needle hub 36. That is, the first needle-tip cover part 70 and the second needle-tip cover part 104 are vertically separated from each other by applying a force, which vertically separates the first and second side plates 50, 52 from each other, to the needle tip protector 10. Then, the inner needle 34 in isolation is inserted between the first and second side plates 50, 52 from the distal end side of the needle tip protector 10, so that the needle tip protector 10 is attached to the inner needle 34. Subsequently, the inner needle hub 36 is fixed to the proximal end part of the inner needle 34 with the needle tip protector 10 attached.

In the present practical embodiment, the needle guide surface 56 is provided to the opening peripheral edge on the distal end side of the needle insertion hole 54 formed in the bottom part 48 of the needle tip protector 10. With this configuration, when inserting the inner needle 34 into the needle insertion hole 54 from the distal end side of the needle tip protector 10, even if the inner needle 34 is slightly deviated in the radial direction with respect to the needle insertion hole 54, the proximal end part of the inner needle 34 comes into abutment against the needle guide surface 56, so that the inner needle 34 is guided into the needle insertion hole 54. Therefore, a high degree of precision in positioning the inner needle 34 and the needle tip protector 10 relative to each other in the radial direction is unlikely to be required, thereby facilitating the attachment operation of the needle tip protector 10 to the inner needle 34.

Besides, the bottom part 48 of the needle tip protector 10 includes the reinforcing tube part 58 protruding from the opening peripheral edge on the proximal end side of the needle insertion hole 54 toward the proximal end side. Since the bottom part 48 is a portion formed from the same single thin-walled plate as the first and second side plates 50, 52, the bottom part 48 is a thin-walled plate, and may be deformed. Therefore, by providing the reinforcing tube part 58, the deformation rigidity of the bottom part 48 can be improved around the needle insertion hole 54. Thus, even if, for example, the proximal end part of the inner needle 34 is abutted against the needle guide surface 56, the deformation of the bottom part 48 is suppressed, thereby effectively obtaining the guiding action of the needle guide surface 56 for the inner needle 34. Additionally, a situation in which the position and shape of the first and second side plates 50, 52 are affected by the deformation of the bottom part 48 is prevented. In the case where the needle guide surface 56 is provided, deterioration in the deformation rigidity of the bottom part 48 due to the needle guide surface 56 can be prevented. It is also possible to provide only the reinforcing tube part 58 without the needle guide surface 56.

By the needle tip protector 10 being attached to the inner needle 34, the first and second needle-tip protection parts 76, 110 of the needle tip protector 10 are vertically separated from each other, and accordingly, the first and second side plates 50, 52 elastically undergo flexural deformation in the plate thickness direction. In the attached state of the needle tip protector 10 to the inner needle 34 before the needle-tip protection operation, the relative inclination angle θ1' between the first proximal end portion 68 of the first side plate 50 and the second proximal end portion 102 of the second side plate 52 is larger than the relative inclination angle θ2' between the first distal end portion 66 and the second distal end portion 100. In the attached state of the needle tip protector 10 to the inner needle 34 before the needle-tip protection operation, the size of the relative inclination angle θ1' between the first proximal end portion 68 and the second proximal end portion 102 is preferably within the range of 0° to 30°. Besides, in the attached state of the needle tip protector 10 to the inner needle 34 before the needle-tip protection operation, the size of the relative inclination angle θ2' between the first distal end portion 66 and the second distal end portion 100 is preferably within the range of -5° to 5°. In the present practical embodiment, the first distal end portion 66 and the second distal end portion 100 are approximately parallel to each other, and the angle θ2' is set to be approximately 0°.

As shown in FIG. 18, with the needle tip protector 10 attached to the inner needle 34, the inner needle 34 is inserted between the first needle-tip stopper part 82 and the second needle-tip stopper part 116. The first needle-tip stopper part 82 is in contact with the outer circumferential surface of the inner needle 34 at the proximal end part of protrusion from the first return piece 80. With this configuration, the contact area between the inner needle 34 and the first needle-tip stopper part 82 is made small, and the frictional resistance when the inner needle 34 is pulled out is reduced. Additionally, the second needle-tip stopper part 116 extends approximately parallel to the needle axis of the inner needle 34, and is in contact with the outer circumferential surface of the inner needle 34 over a wider range in the axial direction than the first needle-tip stopper part 82 is. This effectively exerts the guiding action to guide the inner needle 34 in the needle axis direction when the inner needle 34 is pulled out, thereby suppressing runout (tilting motion) of the inner needle 34 during being pulled out.

As shown in FIGS. 2 to 4, the needle tip protector 10 attached to the inner needle 34 is housed in the lumen of the outer needle hub 20. The distal end portion of the needle tip protector 10 is vertically tapered by the first and second distal-end protection pieces 78, 112 having an inclined shape that mutually approach toward the distal end. Therefore, when inserting the needle tip protector 10 into the outer needle hub 20, it is possible to prevent a situation in which the inner circumferential surface of the outer needle hub 20 is damaged due to unintended catching of the needle tip protector 10 or the like.

Moreover, the length dimension L2 of the first needle-tip cover part 70 is at least 50% of the maximum width dimension W1. With the needle tip protector 10 attached to the inner needle 34 as shown in FIG. 18, the angle α formed by the needle axis of the inner needle 34 and the straight line A connecting the point in the first needle-tip cover part 70 that is located outermost (uppermost) in the direction of mutual facing of the first and second side plates 50, 52 (the vertical direction), namely, the connection point between the first stepped part 74 and the first needle-tip protection part 76, and the point of contact with the inner needle 34 is not greater than 60 degrees. Besides, the length dimension L4 of the second needle-tip cover part 104 is at least 50% of the maximum width dimension W3. With the needle tip protector 10 is attached to the inner needle 34 as shown in FIG. 18, the angle β formed by the needle axis of the inner needle 34 and the straight line B connecting the point in the second needle-tip cover part 104 that is located outermost (lowermost) in the direction of mutual facing of the first and second side plates 50, 52 (the vertical direction), namely, the connection point between the second stepped part 108 and the second needle-tip protection part 110, and the point of contact with the inner needle 34 is not greater than 60 degrees. This allows the lengths of the first needle-tip cover part 70 and the second needle-tip cover part 104 to be sufficiently long in the needle-tip protection state, and the axis-perpendicular component of the force acting on the inner needle 34 due to the elastic force of the needle tip protector 10 can be reduced, thereby decreasing the sliding resistance when pulling out the inner needle 34. Additionally, in the case where the needle tip protector 10 attached to the inner needle 34 is inserted into the radial inside of the outer needle hub 20 or the like, the needle tip protector 10 is unlikely to be pressed strongly against the inner circumferential surface of the outer needle hub 20 due to the displacement of the first and second needle-tip cover parts 70, 104, and damage to the inner circumferential surface of the outer needle hub 20 due to the pressing of the needle tip protector 10 is also prevented.

Furthermore, when the first and second needle-tip cover parts 70, 104 climb over the locking part 42 of the inner needle 34, in the state where the vertically outward displacement of the first and second stepped parts 74, 108 is restricted by the outer needle hub 20, the first and second return pieces 80, 114 are pushed vertically outward by the locking part 42. In the present practical embodiment, the first and second distal-end protection pieces 78, 112 extend while inclining toward the inner needle 34, and the first and second stepped parts 74, 108 and the first and second return pieces 80, 114 are mutually shifted in the needle axis direction. Thus, the relative separation displacement of the first and second return pieces 80, 114 is allowed by the vertically inward displacement of the first and second stepped parts 74, 108 accompanied by the flexural deformation of the first and second side plates 50, 52 (the first and second narrow parts 62, 96) which have low deformation rigidity. Therefore, the vertically outward displacement of the distal end portion of the first and second needle-tip cover parts 70, 104 is easily allowed, and strong pressing of the distal end portion against the inner needle 34 is prevented, thereby decreasing the sliding resistance of the first and second needle-tip cover parts 70, 104 with respect to the inner needle 34.

In addition, the first and second distal-end protection pieces 78, 112 of the first and second needle-tip cover parts 70, 104 extend toward the distal end side while inclining toward the inner needle 34. Accordingly, the positions of the first and second distal-tip cover parts 74, 108 are different in the needle axis direction, and the distal end position of the second needle-tip cover part 104 is located on more distal end side with respect to the proximal end position of the first needle-tip cover part 70. With this configuration, in the needle-tip protection state, the second needle-tip cover part 104 overlaps the first needle-tip cover part 70 with a larger overlap margin, and more stable protection of the needle tip 38 is achieved.

The first and second needle-tip cover parts 70, 104 of the needle tip protector 10 are inserted and housed in the housing part 26 of the outer needle hub 20. Movement of the needle tip protector 10 toward the proximal end side with respect to the outer needle hub 20 is restricted by the first and second stepped parts 74, 108 coming into contact with the inner circumferential surface of the housing part 26. The first and second stepped parts 74, 108 of the needle tip protector 10 are both located on the straight inner circumferential surface 30 of the housing part 26. Thus, the needle tip protector 10 is stably housed in the housing part 26 without only one of the first and second stepped parts 74, 108 being pressed strongly against the outer needle hub 20.

In order to suppress unnecessary rattling against the inner needle 34, the needle tip protector 10 can be arranged such that, for example, the bottom part 48 and the outermost quadrangular parts of the first and second needle-tip cover parts 70, 104 are close to or in contact with the inner circumferential surface of the outer needle hub 20. Besides, in order to limit rotation of the needle tip protector 10 around the inner needle 34, it would also be acceptable that the connecting part 44 of the inner needle hub 36 inserted into the outer needle hub 20 protrudes to the distal end side partially in the circumferential direction (see FIG. 3), and the said protruding portion enters the outside of the side part of the bottom part 48 of the needle tip protector 10.

Regarding the indwelling needle assembly 12 including such a needle tip protector 10, for example, the inner needle 34 and the outer needle 18 puncture a blood vessel or the like of a patient. After puncturing the blood vessel with the inner needle 34 and the outer needle 18, the inner needle 34 is pulled out of the outer needle 18 to the proximal end side by pulling the inner needle unit 16 toward the proximal end side while holding the outer needle unit 14 in the puncture position.

When the inner needle 34 is pulled out of the outer needle 18 to the proximal end side, the needle tip protector 10, which is housed in the housing part 26 of the outer needle hub 20, is restricted in its movement to the proximal end side by the first and second stepped parts 74, 108 getting caught on the proximal tapered inner circumferential surface 32 of the housing part 26. Then, the needle tip protector 10 moves toward the needle tip 38 side while sliding against the outer circumferential surface of the inner needle 34.

At this time, the connection portions between the first and second return pieces 80, 114 and the first and second needle-tip stopper parts 82, 116, which slide against the outer circumferential surface of the inner needle 34, have a curved cross section that expands toward the distal end. Therefore, when pulling out the inner needle 34, the inner needle 34 is unlikely to get caught on the needle tip protector 10 at the connection portion between the first and second return pieces 80, 114 and the first and second needle-tip stopper parts 82, 116. This achieves smooth pulling out of the inner needle 34 to the proximal end side and smooth relative displacement of the needle tip protector 10 to the needle tip 38 side.

With the needle tip protector 10 attached to the inner needle 34 as shown in FIG. 18, the first needle-tip stopper part 82 inclines in the direction of separation from the inner needle 34 toward the distal end of protrusion from the first return piece 80, thereby further reducing the sliding resistance when the inner needle 34 is pulled out. Besides, the connection portions between the first and second return pieces 80, 114 and the first and second needle-tip stopper parts 82, 116, which slide against the outer circumferential surface of the inner needle 34, are shifted with respect to each other in the needle axis direction. Therefore, for example, when the locking part 42 of the inner needle 34, which is larger in diameter than the other portions, passes through the said connection portions, the sliding portions against the inner needle 34 do not climb over the locking part 42 simultaneously but in sequence, thereby reducing the sliding resistance when climbing over the locking part 42.

The front-back length dimension L1 of the first narrow part 62 is within the range of 50% to 100% of the front-back length dimension La of the first side plate 50. The front-back length dimension L3 of the second narrow part 96 is within the range of 50% to 100% of the front-back length dimension Lb of the second side plate 52. With these configurations, the bending deformation rigidities of the first and second narrow parts 62, 96 are made lower.

In the needle tip protector 10 of the present practical embodiment, the sliding resistance is significantly reduced in comparison with the conventional ones. Specifically, regarding a needle tip protector described in FIG. 30 of International Publication No. WO 2023/090462, the sliding resistance value when a large-diameter part corresponding to the locking part 42 of the inner needle 34 passed between a pair of needle-tip cover parts is 160 gf for 16 gauge, 84 gf for 18 gauge, and 64 gf for 24 gauge. Meanwhile, regarding the needle tip protector 10 of the present practical embodiment, the said sliding resistance value is 24 gf for 16 gauge, 16 gf for 18 gauge, and 6 gf or less for 24 gauge. Besides, when a ready-made product of the type in which a side plate crosses an inner needle was measured, the said sliding resistance value for 24 gauge was 30 gf. Since the needle tip protector 10 has such a low sliding resistance value, when the inner needle 34 is pulled out after puncturing the blood vessel, it is possible to prevent a situation in which the outer needle 18 is pulled to the proximal end side together with the inner needle 34 via the needle tip protector 10, thereby preventing unintentional dislodgment of the outer needle 18 from the blood vessel. The dimensions and arrangement of each part of the needle tip protector 10 can be appropriately changed depending on the thickness (the gauge) of the inner needle 34. For example, the positions of the first and second lateral cover pieces 86, 120 and the first and second regulating pieces 92, 126 can be changed depending on the thickness of the inner needle 34.

The sliding resistance of the needle tip protector 10 can be changed by adjusting the length of the first and second narrow parts 62, 96, the length of the connection portion, and the like. Here, the sliding resistance between the first and second needle-tip cover parts 70, 104 and the inner needle 34 when pulling out a 24-gauge inner needle 34 is preferably 25 gf or less, and more preferably 15 gf, for example, 2 to 6 gf. Since the sliding resistance when pulling out the inner needle 34 is set low, the force required to pull out the inner needle 34 can be reduced, thereby making it possible to easily pull out the inner needle 34 with one hand. The maximum outer diameter dimension (the outer diameter dimension at the locking part 42) of the 24-gauge inner needle 34 is 0.531 mm. Therefore, the sliding resistance when the locking part 42 having the outer diameter dimension of 0.531 mm passes between the first and second needle-tip cover parts 70, 104 is preferably 25 gf or less, and more preferably 15 gf, for example, 2 to 6 gf.

By the inner needle 34 being pulled out of the outer needle 18, as shown in FIG. 19, the needle tip 38 of the inner needle 34 is covered by the needle tip protector 10, which has moved to the needle tip 38 side with respect to the inner needle 34. That is, when the inner needle 34 moves toward the proximal end side with respect to the needle tip protector 10 whose movement to the proximal end side is restricted by the outer needle hub 20, the contact between the outer circumferential surface of the inner needle 34 and the first and second return pieces 80, 114 is released, and the first and second side plates 50, 52 elastically recover their shapes. Then, the first and second side plates 50, 52 are deformed to mutually approach toward the distal end, and the first needle-tip cover part 70 covers the distal end side of the inner needle 34, while the second needle-tip cover part 104 is displaced to a position where it covers the distal end side of the first needle-tip cover part 70. This results in a needle tip protection state in which the needle tip 38 of the inner needle 34 is covered by the needle tip protector 10.

In the needle-tip protection state of the needle tip protector 10 as described above, the first distal-end protection piece 78 and the first return piece 80 of the first needle-tip cover part 70 and the second distal-end protection piece 112 and the second return piece 114 of the second needle-tip cover part 104 overlap each other on the distal end side of the needle tip 38.

The pair of first lateral guarding pieces 84, 84 provided to the first needle-tip cover part 70 prevent the needle tip 38 from coming off the first and second needle-tip cover parts 70, 104 in the left-right direction and being exposed to the outside.

In the needle tip protector 10 after the needle-tip protection operation, the first and second needle-tip cover parts 70, 104 approach each other, so that the first and second stepped parts 74, 108 do not engage with the inner circumferential surface of the outer needle hub 20 (the proximal tapered inner circumferential surface 32) in the front-back direction. Therefore, the inner needle 34 is pulled out further toward the proximal end side, and the locking part 42 of the inner needle 34 is locked by the bottom part 48 of the needle tip protector 10 at the peripheral edge of the needle insertion hole 54, so that the needle tip protector 10 moves to the proximal end side integrally with the inner needle 34. Then, by the needle tip protector 10 together with the inner needle 34 being pulled out of the outer needle hub 20 to the proximal end side, the inner needle unit 16 with the needle tip protector 10 attached is separated from the outer needle unit 14.

The outer needle unit 14, which is separated from the inner needle unit 16, is placed as an indwelling needle in a state of puncture into a blood vessel, and functions as an access port to the blood vessel by an external circuit (not shown) being connected to the proximal end side of the outer needle hub 20, and is used, for example, as an administration port for transfusion solution or drug solution, a blood removal port or a blood return port for artificial dialysis, or the like.

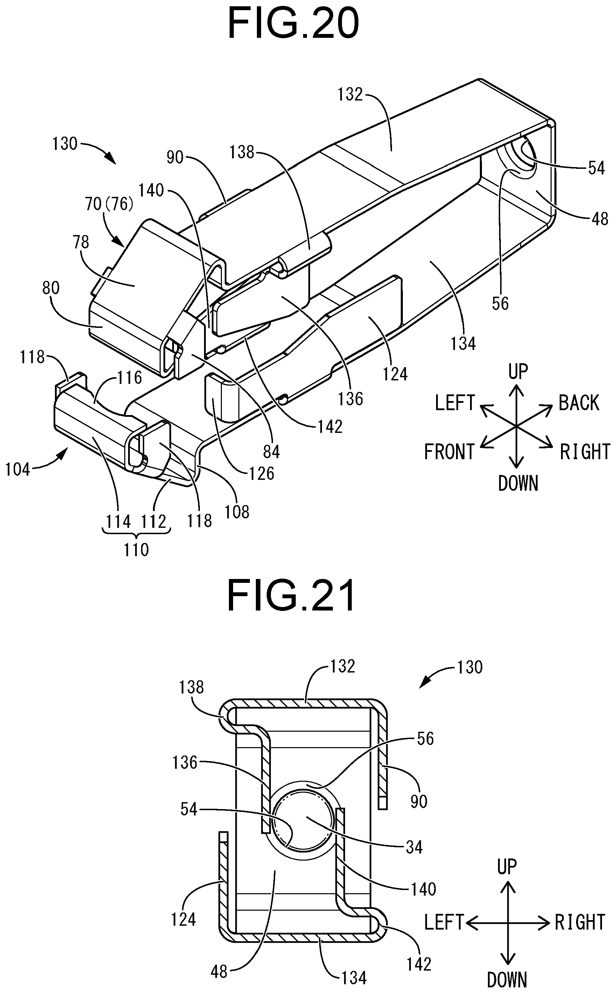

FIGS. 20 and 21 show a needle tip protector 130 according to a second practical embodiment of the present invention. First and second side plates 132, 134 of the needle tip protector 130 do not include the first and second narrow parts 62, 96 like the first and second side plates 50, 52 of the needle tip protector 10 shown in the first practical embodiment, and extend straightly in the front-back direction with an approximately constant left-right width dimension. In the present practical embodiment, the portions of the first and second needle-tip cover parts 70, 104 excluding the first and second lateral guarding pieces 84, 118 also have an approximately constant left-right width dimension, and the first and second side plates 132, 134 and the first and second needle-tip cover parts 70, 104 have approximately the same left-right width dimension as each other. In the following description, components and parts that are substantially identical with those in the first practical embodiment will be assigned like symbols and not described in any detail. The needle tip protector 130 shown in FIGS. 20 and 21 is in the attached state to the inner needle 34, and in the state where the distal end sides of the first and second side plates 132, 134 are displaced in the direction of mutual separation. However, in the isolated state and in the needle-tip protection state, as in the first practical embodiment, the distal end sides of the first and second side plates 132, 134 approach each other, and the first and second needle-tip cover parts 70, 104 overlap each other as viewed in the front-back direction.

A first lateral cover piece 136 protruding toward the second side plate 134 is integrally formed with the first side plate 132 via a first connection portion 138. As shown in FIG. 21, the first connection portion 138 extends toward one side in the left-right direction of the first side plate 132, and then doubles back to extend toward the other side in the left-right direction, and the first lateral cover piece 136 protrudes toward the second side plate 134 from the end of the first connection portion 138. Accordingly, the first lateral cover piece 136 is located on one side in the left-right direction with respect to the needle insertion hole 54, and on the other side in the left-right direction with respect to one side end of the first side plate 132 in the left-right direction.

A second lateral cover piece 140 protruding toward the first side plate 132 is integrally formed with the second side plate 134 via a second connection portion 142. The second connection portion 142 extends toward the other side in the left-right direction of the second side plate 134, and then doubles back to extend toward one side in the left-right direction, and the second lateral cover piece 140 protrudes toward the first side plate 132 from the end of the second connection portion 142. Accordingly, the second lateral cover piece 140 is located on the other side in the left-right direction with respect to the needle insertion hole 54, and on one side in the left-right direction with respect to the other side end of the second side plate 134 in the left-right direction.

With the needle tip protector 130 of this construction according to the present practical embodiment, the first and second lateral cover pieces 136, 140 can be placed close to the lateral sides of the inner needle 34 without providing the first and second narrow parts 62, 96 to the first and second side plates 132, 134, in other words, regardless of the left-right width dimension of the first and second side plates 132, 134, and exposure of the needle tip of the inner needle 34 in the left-right direction can be prevented in the needle-tip protection state. Besides, the connection width dimension (the front-back dimension in FIG. 20) of the first and second connection portions 138, 142 is 70% or less of the maximum width dimension (the front-back dimension in FIG. 20) of the first and second lateral cover pieces 136, 140. While the first and second lateral cover pieces 136, 140 are provided, the formation area of the first and second connection portions 138, 142 in the first and second side plates 50, 52 can be reduced, thereby reducing the area of high rigidity. Accordingly, in comparison with the case where the maximum width dimension of the first and second lateral cover pieces 136, 140 and the connection width dimension of the first and second connection portions 138, 142 are the same, the flexibility of the first and second side plate 50, 52 can be improved, and the resistance value during sliding of the inner needle 34 can be decreased.

While the present invention has been described in detail hereinabove in terms of the practical embodiments, the invention is not limited by the specific description thereof. For example, the left-right width dimension of the first and second narrow parts 62, 96 can be varied in the length direction. The first and second protrusion pieces 90, 124 are not essential, and only one of them may be provided, or neither of them may be provided. It is desirable that the length dimension of the first and second protrusion pieces 90, 124 be large to effectively prevent the needle-tip protection state from being forcibly released by, for example, the left-right widthwise end of the first and second side plates 50, 52 being pushed. However, the length dimension of the first and second protrusion pieces 90, 124 can be not greater than the length dimension of the first and second lateral cover pieces 86, 120.

The specific structure of the first and second needle-tip cover parts 70, 104 is not particularly limited. It would also be possible to provide, for example, a plate-shaped first needle-tip cover part protruding toward the inner needle 34 at the distal end of the first side plate 50, approximately orthogonal to the needle axis direction. The same structure can be adopted for the second needle-tip cover part.

The lateral cover piece may be provided on only one side of the inner needle 34 in the plate width direction (the left-right width direction). Besides, for example, a pair of lateral cover pieces or a pair of protrusion pieces can be provided at the opposite edges in the plate width direction of either one of the first side plate and the second side plate.

At least one of the lateral cover piece and the protrusion piece may be extended to a position close to the bottom part. With this configuration, when, for example, a plurality of needle tip protectors in the isolated state are housed in a box, bottle, etc. for storage, transportation, and the like, the lateral cover piece or the protrusion piece can prevent the needle tip cover part of another needle tip protector from entering the space between the mutually facing pair of side plates.

The preceding practical embodiment illustrates the housing part 26 having an elliptical transverse cross section. However, the housing part may have, for example, an annular shape that is continuous with an approximately constant cross-sectional shape about the entire circumference. In this case, the housing part has an approximately perfect circular shape in cross-section with a diameter larger than the inner diameter of the outer needle hub 20 at a position away from of the housing part.

Moreover, for example, the needle tip protector of the present invention can also be applied to an indwelling needle assembly including a valve structure that connects and blocks the flow path in the outer needle hub, such as that described in International Publication No. WO 2023/145968.

### KEYS TO SYMBOLS

10 needle tip protector (first practical embodiment)
12 indwelling needle assembly
14 outer needle unit
16 inner needle unit
18 outer needle
20 outer needle hub
22 caulking pin
24 screw part
26 housing part
28 distal tapered inner circumferential surface
30 straight inner circumferential surface
32 proximal tapered inner circumferential surface
34 inner needle (puncture needle)
36 inner needle hub
38 needle tip
40 blade surface
42 locking part
44 connecting part
46 filter cap
48 bottom part
50 first side plate (side plate)
52 second side plate (side plate)
54 needle insertion hole
56 needle guide surface
58 reinforcing tube part
60 first wide part
62 first narrow part (narrow part)
64 first intermediate connecting part
66 first distal end portion
68 first proximal end portion
70 first needle-tip cover part (needle-tip cover part)
74 first stepped part
76 first needle-tip protection part
78 first distal-end protection piece
80 first return piece
82 first needle-tip stopper part
84 first lateral guarding piece
86 first lateral cover piece (lateral cover piece)
88 first connection portion (connection portion)
90 first protrusion piece
92 first regulating piece
94 second wide part
96 second narrow part (narrow part)
98 second intermediate connecting part
100 second distal end portion
102 second proximal end portion
104 second needle-tip cover part (needle-tip cover part)
108 second stepped part
110 second needle-tip protection part
112 second distal-end protection piece
114 second return piece
116 second needle-tip stopper part
118 second lateral guarding piece
120 second lateral cover piece (lateral cover piece)
122 second connection portion (connection portion)
124 second protrusion piece
126 second regulating piece
128 molding space
130 needle tip protector (second practical embodiment)
132 first side plate (side plate)
134 second side plate (side plate)
136 first lateral cover piece (lateral cover piece)
138 first connection portion (connection portion)
140 second lateral cover piece (lateral cover piece)
142 second connection portion (connection portion)
L1 length dimension of the first narrow part
L1a length dimension of the first narrow part on the distal end side with respect to the first connection portion
L1b length dimension of the first narrow part on the proximal end side with respect to the first connection portion
La length dimension of the first side plate
L0 entire length of the first side plate and the first needle-tip cover part combined
w1 width dimension of the first narrow part
W1 maximum width dimension of the first needle-tip cover part
L2 length dimension of the first needle-tip cover part
w2 width dimension of the first connection portion in the front-back direction
W2 maximum width dimension of the first lateral cover piece in the same direction
L3 length dimension of the second narrow part
L3a length dimension of the second narrow part on the distal end side with respect to the second connection portion
L3b length dimension of the second narrow part on the proximal end side with respect to the second connection portion
Lb length dimension of the second side plate
L0' entire length of the second side plate and the second needle-tip cover part combined
w3 width dimension of the second narrow part
W3 maximum width dimension of the second needle-tip cover part
L4 length dimension of the second needle-tip cover part
w4 width dimension of the second connection portion in the front-back direction
W4 maximum width dimension of the second lateral cover piece in the same direction
θ1 relative inclination angle between the first proximal end portion and the second proximal end portion in the isolated state
θ2 relative inclination angle between the first distal end portion and the second distal end portion in the isolated state
D vertical distance between facing surfaces of the first side plate and the second side plate
W5 maximum outer dimension of the first needle-tip cover part and the second needle-tip cover part in the vertical direction
θ1' relative inclination angle between the first proximal end portion and the second proximal end portion in the attached state to the needle
θ2' relative inclination angle between the first distal end portion and the second distal end portion in the attached state to the needle
α angle formed by the straight line A and the needle axis of the inner needle
β angle formed by the straight line B and the needle axis of the inner needle

## Claims

1. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation,
needle-tip cover parts are separately provided to respective distal end sides of the pair of side plates while protruding inward in a direction of mutual facing of the pair of side plates,
a narrow part is provided to each of the pair of side plates, the narrow part having a width dimension that is not greater than 70% of a maximum width dimension of the corresponding needle-tip cover part while being flexible in the direction of mutual facing of the pair of side plates, and
a length dimension of the narrow part is at least 50% of a length dimension of the corresponding side plate having the narrow part.

2. The needle tip protector according to claim 1, wherein
in each needle-tip cover part, a length dimension of the needle-tip cover part is at least 50% of the maximum width dimension of the needle-tip cover part, and
in the attached state to the puncture needle, an angle formed by a needle axis of the puncture needle and a straight line, the straight line connecting a point in the needle-tip cover part that is located outermost in the direction of mutual facing of the pair of side plates and a point of contact with the puncture needle, is not greater than 60 degrees.

3. The needle tip protector according to claim 1 or 2, wherein
lateral cover pieces are separately provided to respective widthwise edges of the pair of side plates and protrude inward in the direction of mutual facing, the respective widthwise edges being opposite to each other in a plate width direction, and
a connection portion between the widthwise edge of each of the pair of side plates and the corresponding lateral cover piece has a connection width dimension that is not greater than 70% of a maximum width dimension of the corresponding lateral cover piece in a direction parallel to the connection width dimension.

4. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation,
needle-tip cover parts are separately provided to respective distal end sides of the pair of side plates while protruding inward in a direction of mutual facing of the pair of side plates,
in each needle-tip cover part, a length dimension of the needle-tip cover part is at least 50% of a maximum width dimension of the needle-tip cover part, and
in the attached state to the puncture needle, an angle formed by a needle axis of the puncture needle and a straight line, the straight line connecting a point in the needle-tip cover part that is located outermost in the direction of mutual facing of the pair of side plates and a point of contact with the puncture needle, is not greater than 60 degrees.

5. The needle tip protector according to any one of claims 1-4, wherein respective proximal end parts of a pair of the needle-tip cover parts are located at positions different from each other in a needle axis direction of the puncture needle.

6. The needle tip protector according to claim 5, wherein an outer needle hub on a proximal end side of an outer needle configured to be externally placed about the puncture needle includes a housing part having a concave shape to house the pair of the needle-tip cover parts, and the respective proximal end parts of the pair of the needle-tip cover parts are both located on a straight inner circumferential surface of the housing part.

7. A needle tip protector for preventing exposure of a needle tip by being attached to a puncture needle and moving to a distal end side where the needle tip is located, comprising
a pair of side plates extending along the puncture needle on opposite sides of the puncture needle, wherein
in an attached state to the puncture needle, the puncture needle is inserted between the pair of side plates such that the pair of side plates are elastically deformed to sides of mutual separation,
needle-tip cover parts are separately provided to respective distal end sides of the pair of side plates while protruding inward in a direction of mutual facing of the pair of side plates, and
in an isolated state of the needle tip protector that is not attached to the puncture needle, a distance between facing surfaces of the pair of side plates is set to such a size that prevents entry of the needle-tip cover parts in initial shapes.

8. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation, and
in an isolated state of the needle tip protector that is not attached to the puncture needle, a relative inclination angle between respective proximal end portions of the pair of side plates is smaller than a relative inclination angle between respective distal end portions of the pair of side plates.

9. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation,
lateral cover pieces are separately provided to respective widthwise edges of the pair of side plates and protrude inward in a direction of mutual facing of the pair of side plates, the respective widthwise edges being opposite to each other in a plate width direction, and
a connection portion between the widthwise edge of each of the pair of side plates and the corresponding lateral cover piece has a connection width dimension that is not greater than 70% of a maximum width dimension of the corresponding lateral cover piece.

10. A needle tip protector for preventing exposure of a needle tip by being attached to a puncture needle and moving to a distal end side where the needle tip is located, comprising
a bottom part having a plate shape including a needle insertion hole through which the puncture needle is inserted, wherein
a needle guide surface having an inclination angle is provided to an opening peripheral edge of the needle insertion hole, and a pair of side plates having elasticity are provided from the bottom part toward the distal end side.

11. The needle tip protector according to claim 10, wherein
the needle guide surface is provided to the opening peripheral edge on one side of the needle insertion hole, and
a reinforcing tube part having a tubular shape protrudes from an opening peripheral edge on an other side of the needle insertion hole.

12. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation, and
in the attached state before protection of the needle tip, a relative inclination angle between respective proximal end portions of the pair of side plates is larger than a relative inclination angle between respective distal end portions of the pair of side plates.

13. A needle tip protector for preventing exposure of a needle tip by being attached to a puncture needle and moving to a distal end side where the needle tip is located, comprising:
a pair of side plates extending along the puncture needle on opposite sides of the puncture needle; and
a bottom part connecting proximal end sides of the pair of side plates with each other, wherein
in an attached state to the puncture needle, the puncture needle is inserted through a needle insertion hole penetrating the bottom part, while being inserted between the pair of side plates such that the pair of side plates are elastically deformed to sides of mutual separation,
at least one of the pair of side plates includes a lateral cover piece protruding inward in a direction of mutual facing of the pair of side plates from a widthwise edge of the at least one of the pair of side plates, and
a connection portion between the widthwise edge of the at least one of the pair of side plates and the lateral cover piece has a connection width dimension that is not greater than 70% of a maximum width dimension of the lateral cover piece.
